# EUROPEAN PATENT SPECIFICATION

(11) **EP 4 316 456 B1**
(45) Date of publication and mention of the grant of the patent: **28.10.2026**
(21) Application number: 21933403.4
(22) Date of filing: 28.06.2021
(51) Int. Cl.: A61K 8/18, A61K 8/9789, A61K 33/38, A61K 36/61, A61Q 17/00, A61Q 19/10, A61K 8/19, A61K 8/34, A61K 8/49, A61K 8/92, A61K 31/045, A61K 31/194, A61Q 5/00, C11D 3/382, C11D 3/48, C11D 7/26, C11D 7/44, C11D 17/00

(54) **BIOLOGICALLY ACTIVE QUADROCOMPLEX FOR REGULATING THE BIODIVERSITY OF SKIN MICROBIOTA**
BIOLOGISCH AKTIVER QUADROKOMPLEX ZUR REGULIERUNG DER BIODIVERSITÄT DER HAUTMIKROBIOTA
COMPLEXE À QUATRE COMPOSANTS BIO-ACTIF POUR RÉGULER LA BIODIVERSITÉ DE MICROBIOTES DE LA PEAU

(30) Priority: 26.03.2021 RU 2021108187
(43) Date of publication of application: 07.02.2024
(73) Proprietor: SkyLab AG, Zurich, 8001 (CH)
(72) Inventor: BELOUS, Elena Yur'evna, Moscow, 121309 (RU); FILATOV, Viktor Andreyevich, Chaykovskiy Perm Krai, 617762 (RU)
(74) Representative: GLAWE DELFS MOLL
(86) International application number: PCT/RU2021/000272
(87) International publication number: WO 2022/203538

(56) References cited:
- CN-A- 102 727 399
- CN-A- 107 137 309
- CN-A- 108 210 368
- CN-A- 111 714 543
- EA-A1- 200 700 579
- US-A1- 2006 115 440
- AINANE A. ET AL: "Chemical composition and insecticidal activity of five essential oils: Cedrus atlantica, Citrus limonum, Rosmarinus officinalis, Syzygium aromaticum and Eucalyptus globules", vol. 13, 1 January 2019 (2019-01-01), NL, pages 474 - 485, XP093235449, ISSN: 2214-7853, Retrieved from the Internet <URL:https://pdf.sciencedirectassets.com/305927/1-s2.0-S2214785319X00255/1-s2.0-S2214785319305966/main.pdf?hash=bd5f580373ff3f6bbe09cbdeafe8b3c3475e3e1f31b0eecf8a2cdd25c01ea991&host=68042c943591013ac2b2430a89b270f6af2c76d8dfd086a07176afe7c76c2c61&pii=S2214785319305966&tid=spdf-58d68896-fd4c-4c42-b462-80d> DOI: 10.1016/j.matpr.2019.04.004
- GUY P. P. KAMATOU ET AL: "A Review of the Application and Pharmacological Properties of [alpha]-Bisabolol and [alpha]-Bisabolol-Rich Oils", JOURNAL OF THE AMERICAN OIL CHEMISTS SOCIETY, vol. 87, no. 1, 29 October 2009 (2009-10-29), pages 1 - 7, XP055054582, ISSN: 0003-021X, DOI: 10.1007/s11746-009-1483-3
- ASHOK K DHAKAD ET AL.: "Biological, medicinal and toxicological significance of Eucalyptus leaf essential oil: a review", J SCI FOOD AGRIC, vol. 98, no. 3, 11 September 2017 (2017-09-11), pages 833 - 848, XP055974529, [retrieved on 20180200], DOI: 10.1002/jsfa.8600
- HATICE ZENGIN ET AL.: "Antibacterial and Antioxidant Activity of Essential Oil Terpenes ag ainst Pathogenic and Spoilage-Forming Bacteria and Cell Structure-Activity Relationships Evaluated by SEM Microscopy", MOLECULES, vol. 19, 2014, pages 17773 - 17798, XP055974530, DOI: 10.3390/moleculesl91117773
- MARCEL FORRER ET AL.: "The antimicrobial activity of alpha-bisabolol and tea tree oil against Solobacterium moorei a Gram-positive bacterium associated with halitosis", ARCHIVES OF ORAL BIOLOGY, vol. 58, 2013, pages 10 - 16, XP055974533

## Description

### ART

The invention is set out in the appended set of claims. The present invention can be used to manufacture cosmetic products for hand and/or body care, as well as household cleansers. The biologically active complex is designed for use in cosmetic products for hand and/or body care and/or household cleansers and is comprised of: a) tea tree essential oil (*Melaleuca alternifolia*); b) alpha-bisabolol; c) 1,8-cineol, derived from eucalyptus leaves Eucalyptus spp.; d) aqueous solution of citric acid and silver citrate, where the specified aqueous solution contains 19-23 weight % of citric acid and 2280-2640 parts per million of silver citrate; and where mass ratio of a:b:c:d components makes (0.1-0.5):(0.05-0.3):(0.05-0.35):(0.01-0.2), correspondingly. The quad complex can be used to regulate skin microbiota, including growth inhibition of opportunistic pathogen population, including bacteria, fungi, maintenance of the stable condition of residential microflora, expressed antibacterial effect on transitory microflora, degradation of opportunistic pathogen biofilms.

### PRIOR ART

Every day, a person faces a large number of surfaces in public places and at home. To protect oneself from bacteria, it is necessary to take care about hand hygiene, as it is hand cleanliness which is next to godliness and correspondingly to the high quality of human life. Due to frequent hand contact with the most pathogenic microorganism, in particular, *Staphylococcus aureus, Esherichia coli, Bacillus spp., Enterococcus spp.* and *Pseudomonas aeruginosa,* hygiene remains the most reliable method to protect oneself from bacteria-associated diseases, in conformance with the requirements of the World Health Organization [https://www.who.int/patientsafety/information_centre/Last_April_version HH_Guidelines%5B3%5D.pdf].

According to experts, skin health is one of the fundamental foundations of overall human health. Infectious skin diseases significantly affect the quality of life of people, social aspects and cause the development of other serious diseases. Thus, washing hands with soap significantly reduces the likelihood of developing acute respiratory infections and diarrheal diseases. [Curtis V & Cairncross S (2003) Effect of washing hands with soap on diarrhoea risk in the community: a systematic review. The Lancet Infectious Diseases 3, 275-281.; Rabie T & Curtis V (2006) Handwashing and risk of respiratory infections: a quantitative systematic review. Tropical Medicine& International Health 11, 258-267.; Aiello AE, Coulborn RM, Perez V & Larson EL (2008) Effect of hand hygiene on infectious disease risk in the community setting: a meta-analysis. American Journal of Public Health 98, 1372-1381.]. Despite the widespread use of sanitizers, washing hands with soap afterward remains an important measure to prevent diseases transmitted by the fecal-oral route from the environment through contact with food and water sources. [Curtis V, Cairncross S & Yonli R (2000) Domestic hygiene and diarrhoea - pinpointing the problem. Tropical Medicine&International Health 5, 22-32.]. In addition to diarrheal diseases, handwashing prevents the transmission of pathogens such as pneumonia, influenza, helminthic diseases, trachoma, fetal infections, HIV-associated infections, and gastrointestinal diseases [Aiello AE, Coulborn RM, Perez V & Larson EL (2008) Effect of hand hygiene on infectious disease risk in the community setting: a meta-analysis. American Journal of Public Health 98, 1372-1381; Blencowe H, Cousens S, Mullany LC et al. (2011) Clean birth and postnatal care practices to reduce neonatal deaths from sepsis and tetanus: a systematic review and Delphi estimation of mortality effect. BMC Public Health 11 (Suppl. 3), S11.; Ejemot RI, Ehiri JE, Meremikwu MM & Critchley JA (2012) Hand washing for preventing diarrhoea. Cochrane Database Systematic Review CD004265]. Hand hygiene is a sufficient measure to protect the transmission of infections through food in health care facilities, kindergartens, educational institutions [Roberts L, Jorm L, Patel M, Smith W, Douglas RM & McGilchrist C (2000) Effect of infection control measures on the frequency of diarrheal episodes in child care: a randomized, controlled trial. Pediatrics, 105 (pt 1), 743-746.; Bowen A, Ma H, Jianming O et al. (2007) A cluster-randomized controlled trial evaluating the effect of a handwashing-promotion program in Chinese primary schools. American Journal of Tropical Medicine and Hygiene 76, 1166-1173]. Previous studies confirm that the use of hand hygiene products is one of the most effective measures to prevent global epidemics from both an epidemiological and economic point of view. [Cairncross S & Valdmanis V (2006) Water supply, sanitation, and hygiene promotion. In: Disease Control Priorities in Developing Countries, 2nd edn (eds DT Jamison, JG Breman, AR Measham, G Alleyne, M Claeson, DB Evans, P Jha, A Mills & P Musgrove) World Bank, Washington, DC, Available at: http://www.ncbi.nlm.nih.gov/books/NBK11755/ (Accessed 13 December 2013)].

Hand hygiene is the most ordinary, effective, simple and inexpensive measure to prevent the spread of human health-related infectious diseases (HAIs) [Agência Nacional de Vigilância Sanitária (BR). Higienização das mãos em serviços de saúde [Internet]. [acesso 24 ago 2012]. 2007. Disponivel em: http://bvsms.saude.gov.br/bvs/publicacoes/higienizacao maos.pdf]. Effective hand hygiene not only reduces microbial contamination of the hands like antiseptic agents, but also, when appropriate measures are taken, does not adversely affect the skin. It is recommended that hygiene products be approved by regulatory authorities and be well tolerated by the skin to avoid sensitization and irritation. [Centers for Disease Control and Prevention (USA). Guideline for hand hygiene in health-care settings: recommendations of the Health Care Infection Control Practices Advisory Committee and the HICPAC/SHEA/APIC/IDSA Hand Hygiene Task Force. 2002 [Internet]. [acesso 27 ago 2012] Disponivel em: http://www.cdc.gov/mmwr/preview/mmwrhtml/rr5116a1.htm]. Following the results of meta-analyses and intervention studies worldwide, regular hand hygiene reduces the risk of development of diarrheal diseases by 33% (p<0.05). The use of antibacterial soap can decrease the indicator by 40% [Freeman, M. C., Stocks, M. E., Cumming, O., Jeandron, A., Higgins, J. P. T., Wolf, J., Curtis, V. (2014). Systematic review: Hygiene and health: systematic review of handwashing practices worldwide and update of health effects. Tropical Medicine & International Health, 19(8), 906-916. doi:10.1111/tmi.12339].

Nowadays, there are no one doubts in the need to use hand hygiene products. However, only one third of the world's population washes their hands with soap after using the toilet, and the annual cost of neglecting hygiene rules reaches $30 billion, according to the European Center for Disease Control and Prevention. Washing hands with soap remains the most effective way to prevent infections killing more than 1.4 million children worldwide each year. It has been found that if parents frequently wash their hands, the death rate of newborns from respiratory infections is reduced by 20%, and by 50%.from intestinal infections. Only 19% of people around the world wash their hands after touching contaminated surfaces. One should note that the use of soap for hand washing is influenced by economic well-being of a country. In particular, in low- and middle-income countries, only 13-17% of people wash their hands, correspondingly, while in developed countries this value reaches 42-49%. Residents of the Republic of Korea and Israel have the lowest level of handwashing with soap if compared to highly developed countries of the world. The highest rate of adherence to hygiene was recorded in New Zealand (72%), the UK (52%), the Netherlands (50%) and the USA (49%) [Freeman, M. C., Stocks, M. E., Cumming, O., Jeandron, A., Higgins, J. P. T., Wolf, J., Curtis, V. (2014). Systematic review: Hygiene and health: systematic review of handwashing practices worldwide and update of health effects. Tropical Medicine & International Health, 19(8), 906-916. doi:10.1111/tmi.12339]. Thus, the development of new products with antibacterial effect will support hygiene development strategy in many countries of the world, since low adherence and high incidence of infectious diseases worldwide create the conditions for the emergence of epidemics. According to literature data, the use of soap with an antibacterial effect will eventually reduce the number of deaths by more than 299 thousand people worldwide [Pruss-Ustun A, Bartram J, Clasen T et al. (2014) Burden of diarrhoeal disease from inadequate water, sanitation and hygiene in low- and middle-income settings: a retrospective analysis of data from 147 countries. Tropical Medicine & International Health. doi: 10.1111/tmi.12329]. education of people in the area of hygiene and advice on the use of antibacterial products has reduced the incidence of respiratory infections by 21% in all countries of the world, regardless of the living standards of the population, since the problem of hand hygiene is global [Aiello AE, Coulborn RM, Perez V & Larson EL (2008) Effect of hand hygiene on infectious disease risk in the community setting: a meta-analysis. American Journal of Public Health 98, 1372-1381].

In 2019, over 300 outbreaks of infectious diseases were recorded in the Russian Federation, which occurred as filth-borne diseases. About 85% of those affected are children. These are not only intestinal, but also severe airborne infections. The infectious causative agents are localized in the upper respiratory tract, but also remain for a long time on external objects and human hands. Enteroviruses, rhinoviruses, adenoviruses, influenza viruses persist for a long time on various surfaces, so hand washing remains the most important hygiene procedure that prevents the spread of pathogens and infection of people. Thorough washing of hands with soap reliably removes pathogens off the skin. After washing, about 8% of pathogenic bacteria, such as enterococcus, *Enterococcus spp.,* representatives of coliform bacteria group, *Escherichia spp.,* salmonellas, *Salmonella enterica,* and other bacteria, remain on the skin surface.

It is recommended to wash hands with soap for at least 20 seconds is recommended to prevent infections, but only 5% of people worldwide do this. About one third of people simply rinse their hands with water after using the toilet, and 10% do not wash their hands at all. This conclusion was made by scientists of the University of Michigan (USA) based on observations of 3749 subjects. Before cooking and eating, many people also do not pay attention to the initial cleanliness of their hands. In an experiment by American scientists, only 3% of subjects (out of 383) washed their hands correctly before eating - according to a special technique, while maintaining the time interval. Most subjects washed their hands for less than 20 seconds or did not dry their wet hands with a towel. As a result, pathogenic bacteria capable of causing severe respiratory and intestinal infections were isolated in smears from the palm skin of 81% of the subjects.

Behavioral differences that affect effectiveness of washing hands include the use of gloves, nail polish, jewelry, hand lotion, and adherence to workplace standards. While normal soap works by binding dirt and organic matter, antiseptic soaps contain specific germicidal ingredients that kill both pathogenic and commensal bacteria. One private study showed little difference between hand washing with ordinary toilet soap and with antibacterial soap. Researchers in Korea compared the effects of two soaps on 20 strains of *Escherichia coli, Streptococcus spp.* and *Staphylococcus spp.* It turned out that 0.3% of antibacterial triclosan in soap does not have time to neutralize bacteria in 20 seconds, and some species, for example, *Enterococcus faecalis* and *Staphylococcus aureus,* are better eliminated with ordinary toilet soap. At the same time, antibacterial soap retains a long-lasting antimicrobial effect, and in nine hours, less bacteria were detected on the hand skin than after using ordinary soap, thanks to modifications change in the natural biodiversity of the skin microbiota.

As a result of numerous discussions, the USA Food and Drug Administration issued a decision that over-the-counter antiseptic detergents containing certain active ingredients can no longer be commercialized because manufacturers of such products have not proven that the ingredients are safe for long-term daily use and are more effective than ordinary soap and water in preventing a number of diseases and spreading certain infections. Such ingredients include: Cloflucarban, Fluorosalan, Hexachlorophene, Hexylresorcinol, Iodophors (Iodine-containing ingredients), Iodine complex (ammonium ether sulfate and polyoxyethylene sorbitan monolaurate), Iodine complex (phosphate ester of alkylaryloxy polyethylene glycol), Nonylphenoxypoly (ethyleneoxy) ethanoliodine, Poloxamer-iodine complex, Povidoneiodine 5 to 10 percent, Undecoylium chloride iodine complex, Methylbenzethonium chloride, Phenol (greater than 1.5 percent), Phenol (less than 1.5 percent), Secondary amyltricresols, Sodium oxychlorosene, Tribromsalan, Triclocarban, Triclosan, Triple dye. (https://www.fda.gov/news-events/press-announcements/fda-issues-final-rule-safety-and-effectiveness-antibacterial-soaps). In this regard, consumers' demand in creating effective antibacterial cleanser suitable for processing and applying to the skin of various parts of the body, and that meets modern regulatory standards, is rather relevant.

From an anatomical point of view, human skin is the largest body organ, taking 1/6 of the total body weight, and is involved in maintaining barrier function, protecting against pathogens, chemicals and toxicants, harmful excess UV radiation, temperature fluctuations and dryness. From early ontogenesis, skin is a sterile organ; however, after birth, environmental microorganisms quickly colonize the corneal layer of epidermis with formation of a special bacterial ecosystem, the microbiota. It should be noted that the bacterial cover of skin is unique for each person and changes in different age periods of his life [Dominguez-Bello MG, Costello EK, Contreras M, et al. Delivery mode shapes the acquisition and structure of the initial microbiota across multiple body habitats in newborns. Proc Natl Acad Sci U S A 2010; 107:11971-11975; Capone KA, Dowd SE, Stamatas GN, Nikolovski J. Diversity of the human skin microbiome early in life. J Invest Dermatol 2011; 131:2026-2032.]. According to scientists' estimation, over 100 different types of microorganisms live on the skin, making up a total of 1x10⁷ microorganisms per 1 cm² of human skin [Grice EA, Kong HH, Renaud G, et al. A diversity profile of the human skin microbiota. Genome Res 2008; 18:1043-1050].

Diversity of microbiota is determined by various factors: colonization by non-resident microorganisms, genetic background, lifestyle, demographic characteristics and environmental conditions of the region of residence, as well as compliance with hygiene rules [Ursell LK, Clemente JC, Rideout JR, et al. The interpersonal and intrapersonal diversity of human-associated microbiota in key body sites. J Allergy Clin Immunol 2012; 129:1204-1208; Rosenthal M, Goldberg D, Aiello A, et al. Skin microbiota: microbial community structure and its potential association with health and disease. Infect Genet Evol 2011; 11:839-848.].

According to the theory of P. B. Price, formed in 1938, skin microflora consists of 2 groups: resident microflora, constantly present on the skin, and transient microflora, temporarily present on the surface layers of the skin. The resident microbiota is a normal, commensal microbiota that maintains the homeostasis of other microorganisms and ensures skin health [Chiller K, Selkin BA, Murakawa GJ. Skin microflora and bacterial infections of the skin. J Investig Dermatol Symp Proc 2001; 6:170-174.]. The diversity of the transient and resident microflora of skin is determined by the topographic features of the skin area and its properties (pH, degree of hydration, mineral content and sebum), and can also vary from internal factors (genotype, age and gender) and external individual factors of a person (lifestyle, use of hygienic cosmetic products) [Fierer N, Lauber CL, Zhou N, et al. Forensic identification using skin bacterial communities. Proc Natl Acad Sci U S A 2010; 107:6477-6481]. The function of the resident microflora is to prevent skin colonization with other potentially pathogenic bacteria species, such as *Staphylococcus aureus,* which contribute to the development of inflammatory skin diseases, of dermatological diseases (atopic dermatitis, eczema) and worsen the quality of human life [Iwase T, Uehara Y, Shinji H, et al. Staphylococcus epidermidis Esp inhibits Staphylococcus aureus biofilm formation and nasal colonization. Nature 2010; 465:346-349]. Another important function of resident microorganisms is a modulation of the skin immune system, represented by macrophages, T-lymphocytes and Langerhans cells [Lai Y, Di NA, Nakatsuji T, et al. Commensal bacteria regulate Toll-like receptor 3-dependent inflammation after skin injury. Nat Med 2009; 15:1377-1382; Wanke I, Steffen H, Christ C, et al. Skin commensals amplify the innate immune response to pathogens by activation of distinct signaling pathways. J Invest Dermatol 2011; 131:382-390]. Thus, maintenance of normal microflora is a beneficial factor to ensure the health of human skin [Blaser M. Antibiotic overuse: stop the killing of beneficial bacteria. Nature 2011; 476:393-394.].

The Hygiene Hypothesis, formed in 1989, states that excessive use of harsh detergents and synthetic antiseptic agents in their composition contributes to a more rapid spread of atopic skin diseases due to a pronounced, unnatural change in the biodiversity of the skin microbiota [Strachan DP. Hay fever, hygiene, and household size. BMJ 1989; 299:1259-1260]. At the moment, more and more cases of allergic and chronic inflammatory skin diseases are being detected due to pronounced changes in the skin microbiota with a predominance of transient microflora [Ehlers S, Kaufmann SH. Infection, inflammation, and chronic diseases: consequences of a modern lifestyle. Trends Immunol 2010; 31:184-190]. Damage to relationship between the resident and the transient microflora is one of the driving factors in the development of bacterial inflammatory skin diseases [Kuo IH, Yoshida T, De Benedetto A, Beck LA. The cutaneous innate immune response in patients with atopic dermatitis. J Allergy Clin Immunol 2013; 131:266-278]. It is considered that the presence of *Staphylococcus aureus,* which is found in more than 90% of patients with atopic dermatitis, contributes to the development of the pathogenesis of this dermatological skin disease [Boguniewicz M, Leung DY. Atopic dermatitis: a disease of altered skin barrier and immune dysregulation. Immunol Rev 2011; 242:233-246], and decrease in the colonization of *Staphylococcus aureus* and an increase in resident strains of microflora provides a decrease in the severity of atopic dermatitis in patients [Huang JT, Abrams M, Tlougan B, et al. Treatment of Staphylococcus aureus colonization in atopic dermatitis decreases disease severity. Pediatrics 2009;123: e808-e814]. In a recent study, a resident microorganism, *Staphylococcus epidermidis,* was found to selectively limit the overgrowth of *Staphylococcus aureus* [Iwase T, Uehara Y, Shinji H, et al. Staphylococcus epidermidis Esp inhibits Staphylococcus aureus biofilm formation and nasal colonization. Nature 2010; 465:346-349], and after a decrease in the number of *Staphylococcus aureus,* the number of beneficial species of *Streptococcus spp., Propionebacterium spp.* and *Corynebacterium spp.* genera [Kong HH, Oh J, Deming C, et al. Temporal shifts in the skin microbiome associated with disease flares and treatment in children with atopic dermatitis. Genome Res 2012; 22:850-859]. Thus, elimination of opportunistic microorganisms and safety impact on resident microorganisms allows regulation of biodiversity of microbiota and maintain human skin health.

Hand skin is a place for exchange of microorganisms of the environment and the body. Hands host pathogenic bacterial strains, including methicillin-resistant *Staphylococcus aureus* or *Escherichia coli* [Scott E., Bloomfield S. The survival and transfer of microbial contamination via cloths, hands and utensils. J. Appl. Bacteriol. 1990; 68: 271-278]. Regular use of antiseptic products disrupts the microbiota of hand skin, with a predominance of pathogenic microorganisms. Washing your hands more frequently than necessary removes not only pathogens but also resident microflora. In a private study it was found out that pathogens predominate in people who wash their hands more than 4 times a day [Larson E. Skin hygiene and infection prevention: more of the same or different approaches? Clin. Infect. Dis. 1999; 29: 1287-1294]. Washing hands with soap from refillable open dispensers has been found to increase levels of opportunistic pathogens on the hands of children in primary school [Zapka C., Campbell E., Maxwell S., Gerba C., Dolan M., Arbogast J., Macinga D. Bacterial hand contamination and transfer after use of contaminated bulk-soap-refillable dispensers. Appl. Environ. Microbiol. 2011; 77: 2898-2904]. However, clinical studies have shown a positive effect of washing hands with soap and/or using alcohol-based hand sanitizers in reducing the number of pathogenic bacteria [Fendler E., Ali Y., Hammond B. et al. The impact of alcohol hand sanitizer use on infection rates in an extended care facility. Am. J. Infect. Control. 2002; 30: 226-233; White C., Kolble R., Carlson R. et al. The effect of hand hygiene on illness rate among students in university residence halls. Am. J. Infect. Control. 2003; 31: 364-370].

Since the hands are the main carrier of microorganisms between the environment and the body, the dynamics of microbial communities and the factors that influence them are of great importance for ensuring human health [Lax S., Smith P. Longitudinal analysis of microbial interactions between humans and the indoor environment. Sci. Mag. 2014; 345: 1048]. The hands are rarely considered as a source of beneficial micro-organisms that provide a healthy microflora, highlighting the importance of the hands as a critical vector in microbiome dynamics.

Bacteria make up over 90% of microorganisms of hand skin, while viruses and fungi account for less than 5% [J. Oh, A. Byrd, D. Deming, S. Conlan, H. Kong, J. Segre, Biogeography and individuality shape function in the human skin metagenome, Nature 514 (2014) 59-64.]. In the course of 11 clinical studies, it was found that from 8 to 24 bacteria families, which are combined into 4 large taxonomic groups: *Firmicutes, Actinobacteria, Proteobacteria* (more than 94%) and *Bacteroides* [N. Fierer, M. Hamady, C. Lauber, R. Knight, The influence of sex, handedness, and washing on the diversity of hand surface bacteria, PNAS 105 (2008) 17994- 17999]. The most common microorganisms belong to the families *Staphylococcaceae, Corynebacteriaceae, Propionibacteriaceae, Streptococcaceae.* Representatives of *Corynebacteriaceae* genera *(Actinobacteria* type), in particular, *Corynebacterium xerosis* and *Corynebacterium jeikeium,* are harmless resident microorganisms, being a part of the normal skin microflora that prevents the accumulation of pathogenic microorganisms, and constitute from 2.1 to 23.6% of the microflora of hand skin. Other resident microorganisms *- Staphylococcus epidermidis* and *Staphylococcus hominis (Firmicutes* type) - prevent fixation of pathogenic microorganisms and form protective biofilms, in particular against *Staphylococcus aureus,* and make up from 1.7 to 28.2% of the microflora of hand skin. Also, there are always resident bacteria *Propionibacterium spp.* (*Actinobacteria* type) on the skin the amount from 3.1 to 47.8%, which regulate pH of the acid mantle and secrete natural antimicrobial substances - bacteriocins and propionic acid. *Streptococcus spp.* are presented in an amount from 3.7 to 49%. Thus, the most common types of normal human microflora are gram-positive bacteria of *Staphylococcaceae, Corynebacteriaceae* and *Propionibacteriaceae* families.

Pathogenic and potentially pathogenic species are represented by bacteria of *Bacteroidaceae* (1.6-5.3%), *Enterobacteriaceae* (from 1.2%), *Pseudomonodaceae* (1.1-3.8%), *Porphyromonadaceae* (1.1-2.0%), *Prevotellaceae* (1.0-5.6%), *Flavobacteriaceae* (1.0-10.8%), *Bacillaceae* (1.0-9.7%), *Veillonellaceae* (1.6-5.5%), *Nocardiaceae* (up to 12.4%), *Rhodobacteriaceae* (up to 21.0%) and *Brucellaceae* (up to 4.0%) families, most of which are gram-negative microorganisms. Among fungi, the most common ones are representatives of *Malassezia spp.* and *Aspergillus spp.* genera [K. Findley, J. Oh, J. Yang, S. Conlan, C. Deming, J. Meyer, D. Schoenfeld, E. Nomicos, M. Park, H. Kong, J. Segre, Human skin fungal diversity, Nature 498 (2013) 367-370].

By the moment, a limited number of studies have been presented that estimated the effect of hygiene products on the microbiota of the skin of the hands, which necessitates the study of this topic. One study found that health care workers who regularly use alcohol-based hand sanitizers and report more than 40 cases of washing hands with antibacterial soap per shift had a lower overall biodiversity than the rest of the population [Rosenthal M., Aiello A., Larson E., Chenoweth C., Foxman B. Healthcare workers' hand microbiome may mediate carriage of hospital pathogens. Pathogens. 2014; 3: 1-13]. A time period from the latest handwash affected the biodiversity of the hand microbiota. For example, *Propionibacteria, Neisseriales* and *Pasteurellaceae* were more abundant after washing hands after >2 hours, and *Staphylococcaceae, Streptococcaceae* and *Lactobacillaceae* were more abundant on recently (<2 hours) washed hands [Fierer N., Hamady M., Lauber C., Knight R. The influence of sex, handedness, and washing on the diversity of hand surface bacteria. PNAS. 2008; 105: 17994-17999]. Changes in the biodiversity of the hand skin microbiota have been observed over time since the last handwash. One should especially note that the use of oral antibiotics had a significant impact on the hand microbiome, with the largest shift observed around the time of use [Flores G., Caporaso G., Henley J., Rideout J., Domogala D., Chase J., Leff J., Vázquez-Baeza Y, Gonzalez A., Knight R., Dunn R., Fierer N. Temporal variability is a personalized feature of the human microbiome. Genome Biol. 2014; 15: 531].

Significant differences are also based on a person's lifestyle. People living in the same house have a more similar hand microbiome than people living in different houses; and couples and their young children have more bacterial taxa than unrelated roommates [Lax S., Smith P. Longitudinal analysis of microbial interactions between humans and the indoor environment. Sci. Mag. 2014; 345: 1048]. Moreover, having a pet led to a significant increase in opportunistic pathogens on the skin, and human hand microbiota has become more similar to their own pet's paws than to the paws of a pet in another household. In general, having a pet increases the overall diversity of bacteria on hands [Song S., Lauber C., Costello E., Lozupone C., Humphrey G., Berg-Lyons D., Knight R. Cohabiting family members share microbiota with one another and with their dogs. eLife. 2013; 2: e00458].

Interaction with inanimate objects is another source of variability in hand microbiota. A home is colonized by the microbiota of its occupant, and for most hand samples, microbial biodiversity can be compared to light switches in homes, indicating that hands are the key vector of microbial contamination of surfaces inside the home [Lax S., Smith P. Longitudinal analysis of microbial interactions between humans and the indoor environment. Sci. Mag. 2014; 345: 1048].

To protect against overgrowth of pathogenic microorganisms and maintain homeostasis of the resident microflora on skin, it is necessary to use hygiene products with antibacterial substances that do not disturb the populations of human microorganisms. But at the moment there is the problem of the presence of superbugs - bacteria that are resistant to most of the antibacterial agents used by mankind. The prerequisites for antibiotic resistance were the and uncontrolled use of synthetic and semi-synthetic antibacterial substances in the composition of liquid soap and solid bar soap, household cleansers and oral care products. According to the Food and Drug Administration (FDA), the use of triclosan, triclocarban, chlorooxylenol and benzalkonium chloride is possible after evaluating the safety and effectiveness of the components in long-term use [https://www.fda.gov/consumers/consumer-updates/antibacterial-soap-you-can-skip-it-use-plain-soap-and-water]. The effectiveness of these components may decrease with prolonged use, since bacteria of hand skin develop resistance to them, which directly affects the safety of products with these components and, in general, determines the need for their use. Additionally, 19 more components require an additional safety assessment referring to regular use in disinfectants and cosmetics [https://www.federalregister.gov/documents/2016/09/06/2016-2133 7/safety-and-effectiveness-of-consumer-antiseptics-topical-antimicrobial-drug-products-for].

An important parameter to evaluate the antibacterial action of the components is the ability to prevent the formation of biofilms of resistant bacteria together with the resident microflora. Microbiota is a harmonious system, so it is important to maintain a balance of microorganisms both on the skin and on surfaces. In particular, *Staphylococcus aureus,* together with other opportunistic microorganisms, is able to form stable biofilms from the exopolysaccharide matrix and contribute to the development of dermatological diseases, in particular atopic dermatitis. It should be noted that not all antiseptic gels and antibacterial hand soaps show a pronounced antibacterial effect due to the prevalence of bacterial biofilms on the surface of the skin of the hands. Even products based on ethyl alcohol, isopropyl alcohol and triclocarban show little antibacterial effect in in vitro studies on the inhibition zone [Khalid Ahmed, Hamza Ahmed, Fasih Ali Ahmed, Alisha Akbar Ali, Jehangir Akbar, Junaid Rana, Uroosa Tariq, Syed Hani Abidi. Analysis of anti-microbial and anti-biofilm activity of hand washes and sanitizers against S. aureus and P. aeruginosa. J Pak Med Assoc. 2020 Jan;70(1):100-104. doi: 10.5455/JPMA.2776.]. This is due to the fact that the reference bacteria, *Staphylococcus aureus* and *Pseudomonas aeruginosa* are the most resistant microorganisms due to genetic rearrangements and the formation of protective biofilms as one of the pathogenicity factors of bacteria. Thus, there is a need for an antibacterial complex that could destroy biofilms of pathogenic microorganisms on the skin and contaminated surfaces, penetrate deeply into bacterial colonies, destroy opportunistic microorganisms without developing resistance in them, while changing biodiversity towards resident microorganisms.

According to the statement by Margaret Chan, the head of the World Health Organization, the world is at the stage of the post-antibiotic era [https://biomolecula.ru/articles/evoliutsiia-naperegonki-ili-pochemu-antibiotiki-perestaiut-rabotat]. The most affordable way to stop the growth of antibiotic resistance can be the use of essential oils and the main biologically active components of them. Scientific studies confirmed that many types of essential oils and their main components have an expressed antimicrobial activity, and their combinations are effective in combating pathogenic microorganisms without inhibiting populations of resident microorganisms.

Tea tree essential oil has a number of well-known antiseptic properties [Carson CF, Hammer KA, Riley TV. Melaleuca alternifolia (Tea Tree) Oil: a review of antimicrobial and other medicinal properties. Clin Microbiol Rev. 2006;19(1):50-62]. According to a scientific review, this essential oil not only reduces the colonization of methicillin-resistant Staphylococcus aureus on surfaces, but also has antifungal [Hammer KA, Carson CF, Riley TV. Antifungal activity of the components of Melaleuca alternifolia (tea tree) oil. J Appl Microbiol. 2003;95(4):853-900] and anti-inflammatory effects [Brand C, Ferrante A, Prager RH, Riley TV, Carson CF, Finlay-Jones JJ, et al. The water-soluble components of the essential oil of Melaleuca alternifolia (tea tree oil) suppress the production of superoxide by human monocytes, but not neutrophils, activated in vitro. Inflamm Res. 2001;50(4):213-9].

The mechanism of the antibacterial action of this essential oil is explained by the chemical components of terpenoid structure and the lipophilicity of the components. The components of the essential oil help to increase the permeability of bacterial cell membranes due to the formation of liposomal structures [Cox, S. D., C. M. Mann, J. L. Markham, H. C. Bell, J. E. Gustafson, J. R. Warmington, and S. G. Wyllie. 2000. The mode of antimicrobial action of the essential oil of Melaleuca alternifolia (tea tree oil). J. Appl. Microbiol. 88:170-175], which explains an increase in ion permeability and causes cell lysis. Additionally, essential oil components block the action of respiratory enzymes, bind K + potassium ions and increase the sensitivity of bacteria to sodium chloride and preservatives [C. F. Carson, K. A. Hammer, and T. V. Riley. Melaleuca alternifolia (Tea Tree) Oil: a Review of Antimicrobial and Other Medicinal Properties. Clinical Microbiology Reviews, Jan. 2006, Vol. 19, No. 1, p. 50-62. doi:10.1128/CMR.19.1.50-62.2006].

Moreover, tea tree essential oil, due to the high content of terpinen-4-ol (over 38%), fights against the most resistant strains of bacteria, in particular *Staphylococcus aureus* [Loughlin, B.F. Gilmore, P.A. McCarron, M.M. Tunney. Comparison of the cidal activity of tea tree oil and terpinen-4-ol against clinical bacterial skin isolates and human fibroblast cells, Lett. Appl. Microbiol. 46 (2008) 428]. There is data evidencing that tea tree essential oil prevents influenza viruses from entering host cells by disrupting the integrity and blocking envelope enzymes, which helps preventing viral replication and disease progression [Li X, Duan S, Chu C, Xu J et al (2013) Melaleuca alternifolia Concentrate Inhibits in Vitro Entry of Influenza Virus into Host Cells Molecules, 18: 9550-66]. In a clinical study, liquid soap with 0.3% tea tree essential oil was shown to be more effective against hand skin pathogens than 0.3% triclosan [Gnatta JR, Pinto FMG, Bruna CQM, Padoveze MC, Graziano KU, Silva MJPD. 2015. Comparison of hand hygiene antimicrobial efficacy: Melaleuca alternifolia essential oil versus triclosan versus chlorhexidine. Antimicrob Resist Infect Control 4: 1212-1219].

In vitro studies demonstrated that tea tree essential oil has a more pronounced effect against pathogenic and opportunistic microorganisms than against resident representatives of the normal microbiota. The minimum inhibitory concentration (MIC) against pathogenic bacteria is 1.0% or less, while being more than 2% against commensal bacteria of *Staphylococcus spp.* and *Micrococcus spp.* genera [C. F. Carson, K. A. Hammer, and T. V. Riley. Melaleuca alternifolia (Tea Tree) Oil: a Review of Antimicrobial and Other Medicinal Properties. Clinical Microbiology Reviews, Jan. 2006, Vol. 19, No. 1, p. 50-62. doi:10.1128/CMR.19.1.50-62.2006].

Thus, there is data on the minimum concentration of tea tree essential oil to inhibit the growth of the following pathogens associated with diseases of skin, gastrointestinal tract and respiratory system: *Bacillus cereus* (0.3%), *Bacteroides spp.* (0.06-0.50%), *Enterococcus faecalis* (>0.5%), *Escherichia coli* (0.08-2.00%), *Klebsiella pneumoniae* (0.25-0.30%), *Pseudomonas aeruginosa* (1-8%), methicillin-resistant *Staphylococcus aureus* (0.04-0.35%), *Streptococcus pyogenes* (0.12-2.00%). It should be noted that these values are higher for representatives of normal resident microflora: *Corynebacterium spp.* (>2%), *Micrococcus luteus* (0.25-6%), *Lactobacillus spp.* (1-2%), *Staphylococcus epidermidis* (>4%) and *Staphylococcus hominis* (4%). Thus, tea tree essential oil has a targeted antibacterial activity against pathogenic microorganisms, rather than representatives of the normal skin microflora.

1,8-cineol (formerly eucalyptol) is the main component of the essential oil of *Eucalyptus globulus,* known for its antibacterial and antiviral properties [Dhakad, A. K., Pandey, V. V., Beg, S., Rawat, J. M., & Singh, A. (2017). Biological, medicinal and toxicological significance of Eucalyptus leaf essential oil: a review. Journal of the Science of Food and Agriculture, 98(3), 833-848. doi:10.1002/jsfa.8600]. According to the chemical structure, 1,8-cineol is menthane oxide, a monocyclic terpene with an oxygen fragment, due to which it has the ability to penetrate through hydrophobic membranes of various nature. The lipophilic nature of monoterpene determines the fact that it is predominantly transferred from aqueous phase into membrane structures, which leads to an increase in membrane permeability, an increase in its fluidity, a disruption in the functioning of proteins built into the membrane, and a change in the processes of transfer of ions and molecules (osmotic balance) into bacterial cells [Sikkema, J.; de Bont J.A.M.; Poolman, B. Interactions of cyclic hydrocarbons with biological membranes. J. Biol. Chem. 1994, 269, 8022-8028; Sikkema, J.; de Bont J.A.M.; Poolman, B. Mechanisms of membrane toxicity of hydrocarbons. Microbiol. Rev. 1995, 59, 201-222.].

It was found out that the combination of 1,8-cineol and tea tree essential oil makes it possible to reduce the concentration of the components by 2 times while maintaining the high efficiency of the combination in protecting against *Salmonella typhimurium, Escherichia coli* and *Staphylococcus aureus* bacteria [Zengin H, Baysal AH. Antibacterial and antioxidant activity of essential oil terpenes against pathogenic and spoilage-forming bacteria and cell structure-activity relationships evaluated by SEM microscopy. Molecules. 2014 Nov 3;19(11):17773-98. doi: 10.3390/molecules191117773]. It has been established that terpinen-4-ol, the main component of tea tree essential oil (>40%), does not cause pronounced autolysis of resistant *Staphylococcus aureus,* and the combination with 1,8-cineol provides a high antibacterial effect due to an increase in the permeability of bacterial biofilms and membranes. So, 1,8-cineol acts as a conductor, facilitating a deeper penetration of the components into the bacteria.

Thus, this substance is a penetration enhancer, i.e., it enhances the penetration of antibacterial substances through biofilms, cell walls and bacterial membranes, and also prevents the formation of biofilms of microorganisms when used together. 1,8-cineol in combination with antibacterial agents (terpinen-4-ol, alpha-terpineol, chlorhexidine bigluconate, etc.) has an effective antibacterial effect [Williams AC, Edwards HGM, Lawson EE et al. Molecular interactions between the penetration enhancer 1,8-cineole and human skin. J Raman Spectrosc 2006; 37: 361-6.]. Since cell walls and membranes of gram-positive and gram-negative bacteria differ in chemical composition, the use of 1,8-cineol to increase the permeability of antibacterial components is one way to target it to increase penetration and, as a result, antibacterial effect.

Also alpha-bisabolol is a sesquiterpene alcohol, the main component of the candeia essential oil, *Vanillosmopsis erythropappa,* chamomile essential oil, *Chamomilla recutita,* and lavender essential oil, *Lavandula angustifolia.* The substance has a pronounced anti-inflammatory and antibacterial activity against microorganisms of *Staphylococcus aureus, Bacillus cereus, Shigella shiga, Shigella sonnei, Pseudomonas aeruginosa* and *Proteus spp* [Kazemi M. Chemical composition and antimicrobial activity of essential oil of Matricaria chamomilla. Bull. Env. Pharmacol. Life Sci., Vol (3) January 2014: 148-153]. A high antibacterial activity is explained by the amphiphilic nature of the component: the presence of double bonds that ensure penetration through the lipid membrane, and alcohol hydroxyl, which interacts with water and promotes the formation of pores for substance penetration, and also inhibits enzyme systems of microorganisms. Alpha-bisabolol impacts unformed bacterial biofilms, and when used together with penetration enhancers, it has an effective antibacterial effect [Robyn L. van Zyl. Sammy T. Seatlholo and Sandy F. van Vuuren. The Biological Activities of 20 Nature Identical Essential Oil Constituents. J. Essent. Oil Res., 18, 129-133].

Another direction in skin hygiene is the use of metal ions, to which resistance is not developed, and its antibacterial effect is stable. Silver ions have a long history, as they were used to treat bacterial infections and heal wounds during military operations. It turned out that unlike most medicinal substances bacteria practically do not develop antibiotic resistance to silver ions [Simon Silver. Bacterial silver resistance: molecular biology and uses and misuses of silver compounds. FEMS Microbiology Reviews, Volume 27, Issue 2-3, June 2003, Pages 341-353, https://doi.org/10.1016/S0168-6445(03)00047-0]. Mechanisms with which silver ions exert their effect on bacteria and viruses have been remained unclear for a long time. However, scientists from the University of Arkansas studied the effect of silver ions on all molecular targets of a bacterial cell [https://aem.asm.org/content/86/6/e02479-19/article-info]. When added to *Escherichia coli* bacteria, silver ions weakened the bonds between proteins and DNA molecules, which led to a disruption in the growth of bacteria and their subsequent death. Silver ions also cause oxidative stress in cells [Xiangwen Liao, Fang Yang, Hongyan Li, Pui-Kin So, Zhongping Yao, Wei Xia, Hongzhe Sun. Targeting the Thioredoxin Reductase-Thioredoxin System From Staphylococcus Aureus by Silver Ions. Inorg Chem. 2017 Dec 18;56(24):14823-14830. doi: 10.1021/acs.inorgchem.7b01904. Epub 2017 Nov 28], modify the structure of protein molecules and bacterial cell membranes [Anna Kedziora, Mateusz Speruda, Eva Krzyzewska, Jacek Rybka, Anna Lukowiak, Gabriela Bugla-P 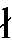 oskońska. Similarities and Differences Between Silver Ions and Silver in Nanoforms as Antibacterial Agents. Int J Mol Sci. 2018 Feb 2;19(2):444. doi: 10.3390/ijms19020444]. Silver ions have been found to prevent overgrowth of bacteria and formation of dangerous biofilms on surfaces, including antibioticresistant bacteria. In March 2020, the U.S. Federal Government's Environmental Protection and Health Agency (EPA) published a list of substances that fight SARS-CoV-2 coronavirus. Beside alcohols, quaternary ammonium compounds, hydrogen peroxide and sodium hypochlorite, it also includes silver ions [https://www.epa.gov/pesticide-registration/list-n-disinfectants-use-against-sars-cov-2]. Scientific evidence confirms that silver particles have antiviral properties against HIV-1 [https:/ /pubs.rsc.org/en/content/articlelanding/2005/CC/b51 0984a#! div Abstract], hepatitis B virus [Lei Lu, Raymond Wai-Yin Sun, Rong Chen, Chee-Kin Hui, Chi-Ming Ho, John M Luk, George K K Lau, Chi-Ming Che. Silver Nanoparticles Inhibit Hepatitis B Virus Replication. Antivir Ther. 2008;13(2):253-62], respiratory syncytial virus [P L Taylor, O Omotoso, J B Wiskel, D Mitlin, R E Burrell. Impact of Heat on Nanocrystalline Silver Dressings. Part II: Physical Properties. Biomaterials. 2005 Dec;26(35):7230-40. doi: 10.1016/j.biomaterials.2005.05.041] and type 1 herpes simplex virus [https://nanoscalereslett.springeropen.com/articles/10.1007/s11671-008-9128-2].

An important parameter to evaluate antibacterial action of the components is the ability to prevent the formation of biofilms of resistant bacteria together with the microflora. A microbiota is a harmonious system, so it is important to maintain a balance of microorganisms both on the skin and on surfaces. In particular, *Staphylococcus aureus,* together with other opportunistic microorganisms, is able to form stable biofilms from the exopolysaccharide matrix and contribute to the development of dermatological diseases, in particular atopic dermatitis. It should be noted that not all antiseptic gels and antibacterial hand soaps show a pronounced antibacterial effect due to the prevalence of bacterial biofilms on the surface of the skin of the hands. Even products based on ethyl alcohol, isopropyl alcohol and triclocarban show little antibacterial effect in in vitro studies on the inhibition zone. This is due to the fact that the reference bacteria, *Staphylococcus aureus* and *Pseudomonas aeruginosa,* are the most resistant microorganisms due to genetic rearrangements and the formation of protective biofilms as one of the pathogenicity factors of bacteria. Thus, there is a need for a complex of components that could destroy biofilms of pathogenic microorganisms on skin and contaminated surfaces, penetrate deeply into bacterial colonies, and destroy opportunistic microorganisms without developing resistance in them.

Patent Application US20110244030A1 [MEDICA AVENUE (USA)] published on October 06, 2011, discloses compositions and method for topical application. The composition contains a foaming component and a carrier, as well as at least one active component. The foaming agent may be a cationic, anionic, amphoteric, or nonionic surfactant. The active ingredient may include eucalyptus essential oil, eucalyptol, chamomile extract, chamomile essential oil, tea tree essential oil, or silver particles. The observations of the authors demonstrate that compositions with this formulation have the highest bioavailability. The embodiment may be a lotion, gel or other liquid form, as well as a topical antibacterial aerosol.

The compositions described above from Patent Application US20110244030A1 have a number of components not preferrable for frequent use in products that come into direct contact with hand skin. Anionic and cationic surfactants are sufficiently effective; however, the charged residues of the molecules cause an aggressive effect on hand skin, in particular, increased sensitivity of hands with minimal contact and complete destruction of microflora. Antibacterial components are used separately, due to with which the risk of the emergence of multi-resistant microorganisms increases.

Hand hygiene spray "75% Alcohol & Silver Hand Sanitizing Spray" (<UPL:https://www.skinprobiotics.net/products/70-alcohol-silver-hand-cleansing-gel?variant=31481204441145>) is known, containing the following components: 75% isopropyl alcohol (w/w), aloe vera juice, vegetable glycerin, hydrogen peroxide, tea tree essential oil, eucalyptus essential oil, citric acid, 10+ ppm silver ions.

The composition described above is based on 75% isopropyl alcohol, which is a fast-acting antiseptic that removes all microflora from the surface of the skin of the hands. The absence of favorable microflora after the use of alcohol solutions contributes to rapid colonization by pathogenic microorganisms. Tea tree essential oil and eucalyptus are aromatic compositions, since the main antibacterial effect is determined by isopropyl alcohol. The active concentration of Ag+ silver ions for a stable antibacterial effect is 2000-2500 ppm, so in this case the component performs a marketing function.

After shave cream "Beard Club After Shave Cream" (<URL: https://1only.by/ukhodza-kozhej/15649-krem-posle-britya-beard-club>) is known, containing the following components (INCI names are specified): Aqua (Water), Dicaprylyl Ether, Polyquaternium-37, Eucalyptol, Eucalyptus Globulus Leaf Oil, Rosmarinus Officinalis (Rosemary) Leaf Oil, Pinus Sylvestris Leaf Oil, Glycyrrhetinic Acid, Lavandula Angustifolia (Lavender) Oil, Citrus Aurantium Bergamia (Bergamot) Leaf Oil, Citrus Medica Limonum (Lemon) Peel Oil, Bisabolol, Cupressus Sempervirens Oil, Myrtus Communis Oil, Isobornyl Acetate, Origanum Majorana Leaf Oil, Origanum Vulgare Oil, Melaleuca Alternifolia (Tea Tree) Leaf Oil, Potassium Azeloyl Diglycinate, Thymus Vulgaris Oil, Borneol, Pelargonium Graveolens Flower Oil, Citrus Aurantium Dulcis (Orange) Peel Oil, Guaiazulene, Citrus Nobilis (Mandarine Orange) Peel Oil, Citrus Grandis (Grapefruit) Peel Oil, Eugenia Caryophyllus Leaf Oil, Cymbopogon Martini Oil, Citrus Aurantium Amara (Bitter Orange) Peel Oil, PEG-40 Hydrogenated Castor Oil, PPG-26-Buteth-26, Salicylic Acid, Eugenol, Limonene, Linalyl Acetate, Linalool, Citral, Citronellol.

The composition described above contains eucalyptol, bisabolol, tea tree essential oil and other components for an anti-inflammatory effect, since skin shaving is accompanied by irritation and redness. An additional antibacterial effect is mediated by the combination of essential oils. However, this composition is not intended for hand skin hygiene and maintaining the balance of microflora. Due to a wide range of essential oils containing cosmetic allergens (eugenol, limonene, linalyl acetate, linalool, citral and citronellol) and salicylic acid in the composition, it can cause unpleasant dermatological sensations and has the risk of causing an allergic reaction in people with sensitive skin, which does not provide dermatological comfort to consumers.

Cosmetic soap "Intimate Soap Camomile, Calendula & Tea Tree" (<URL:https://www.ecco-verde.com/essentiq-for-you-from-nature/intimate-soap-camomile-calendula-tea-tree>), is known, containing the following components (INCI names are specified): Aqua (Water), Lauryl Glucoside, Aloe Barbadensis Leaf Juice, Lavandula Angustifolia (Lavender) Flower Water, Cocamidopropyl Betaine, Coco Glucoside, Glyceryl Oleate, Chamomille Recutita (Matricaria) Flower Extract, Calendula OffIcinalis (Calendula) Flower Extract, Salvia Officinalis Leaf Extract, Capryloyl/caproyl Methyl Glucamide, Lauroyl/myristoyl Methyl Glucamide, Sodium Cocoyl Hydrolyzed Amaranth Protein, Lactic Acid, Citric Acid, Lavandula Angustifolia (Lavender) Flower Oil, Citrus Paradisi (Grapefruit) Peel Oil, Melaleuca Alternifolia Leaf Oil, Dehydroacetic Acid, Potassium Sorbate, Benzyl Alcohol, Linalool, Limonene, Geraniol, Citral, Citronellol.

The cosmetic product described above is intended for intimate area hygiene, not for hand skin. Considering the fact that the microflora of the mucous membranes of the intimate areas and the hand skin have significant differences in qualitative and quantitative content, the composition cannot be used for hand skin hygiene in order to prevent antibacterial manifestations. Tea tree essential oil acts as a fragrance in this composition, since lactic and citric acids have the main effect as mild antibacterial regulators of the microflora of the intimate area.

Joining the components in the present invention achieves synergetic effects and maintains efficiency at a lower percentage of input of each component. An innovative complex, including tea tree essential oil *Melaleuca alternifolia oil,* bisabolol, 1,8-cineol and silver ions, is designed for protection against opportunistic pathogens on the skin and surfaces through an unexpectedly effective penetration deep into biofilms, which leads to the death of bacterial cells. The complex does not cause the development of resistance in bacteria, which allows it to be regularly used as part of cosmetic products for everyday use, household cleansers, disinfectants, etc.

The proposed innovative composition contains a synergetic combination of active ingredients, demonstrating an unexpected supertotal effect against pathogenic and/or opportunistic microorganisms, with a decrease in their biodiversity in general, while maintaining beneficial microflora, for example, on the skin. Without being bound to any theory, the inventors believe that the main effect is to inhibit enzyme systems and change the structure of protein molecules, weakening bonds between DNA strands and the appearance of oxidative stress, followed by disruption of bacterial growth and preventing the formation of stable biofilms on various surfaces. There is a deeper penetration of antibacterial agents through biofilms and even inside bacteria; an enhancer effect is observed, as well as increase in the antibacterial activity of the components, which allows them to be used in low concentrations, as well as to minimize the risk of developing resistance in bacteria. By increasing the permeability of the bacterial cell membrane, increasing its fluidity, disrupting the functioning of proteins built into the membrane, as well as changing the processes of transfer of ions and molecules, osmotic stress occurs, which causes the death of bacterial colonies and further increases the penetration of antibacterial agents. The formation of liposomal structures may occur, further violating the integrity of the bacterial cell membrane and territorially delimiting bacterial elements, including quorum sensing molecules (QS molecules). As a result of application, the composition for to the invention promotes the formation of a protective film on a surface, such as, for example, skin surface, for long-term protection and maintenance of hygiene. Thus, the combined use of the components of the composition according to the invention leads to a powerful increase of the antibacterial action due to the suppression of all major molecular targets and, subsequently, the vital activity of bacteria, and also makes it possible to ensure the homeostasis of the microbiota, for example, of hand skin. A distinctive feature is that the components in the claimed concentrations act only against pathogenic and opportunistic microorganisms, which allows stimulating the growth of resident microflora for natural protection. Also, there is no need to introduce aggressive synthetic antibacterial substances into the composition of the complex, such as triclosan, triclocarban, benzalkonium chloride, cetylpyridinium chloride and ethyl alcohol.

The achievement of the claimed technical results is illustrated in detail in the Experimental of this document. Such achievable technical results will be readily understood by those skilled in the art based on the present research data. In addition, the possibility of using the quad complex for the invention to regulate the skin microbiota, including inhibition of the growth of the population of opportunistic microorganisms, including bacteria, fungi, maintaining a stable state of the resident microflora, a pronounced antibacterial effect on the transient microflora, the destruction of biofilms of opportunistic pathogens, is noted.

The present invention is defined in the claims and relates to cosmetic products and/or household cleansers. Compositions for personal care and home care are known from prior art, e.g. US 2006/0115440 A1.The present invention as defined in the claims differs from US 2006/0115440 A1, which can be considered as the closest prior art, in the addition of a combination of tea tree essential oil, alpha-bisabolol, 1,8-cineol in combination with aqueous silver citrate in ratios of 0.1-0.5:0.05-0.3:0.05-0.35:0.01-0.2, respectively. The advantageous technical effects of said distinguishing features are outlined in detail in the present specification and have been shown in terms of hard data exemplarily in Example 3.

### SUMMARY OF THE INVENTION

The invention is set out in the appended set of claims. In the first aspect, the invention relates to the biologically active complex, designed for use in cosmetic products and/or household cleansers, comprising of:
a) tea tree essential oil (*Melaleuca alternifolia*),
b) alpha-bisabolol,
c) 1,8-cineol, derived from eucalyptus leaves Eucalyptus spp.,
d) aqueous solution of citric acid and silver citrate,
where the specified aqueous solution contains 19-23 weight % of citric acid and 2280-2640 parts per million of silver citrate; and where mass ratio of a:b:c:d components makes (0.1-0.5):(0.05-0.3):(0.05-0.35):(0.01-0.2), correspondingly.

In the second aspect, the invention relates to a liquid soap, containing 0.3-0.7 weight % of the biologically active complex for the invention.

In the third aspect, the invention relates to a shower gel, containing 0.3-0.7 weight % of the biologically active complex for the invention.

In the fourth aspect, the invention relates to a scalp care product, containing 0.3-0.7 weight % of the biologically active complex for the invention.

In the fifth aspect, the invention relates to a body care product, containing 0.3-0.7 weight % of the biologically active complex for the invention.

In the sixth aspect, the invention relates to a cosmetic hygienic product without ethyl alcohol, containing 0.3-0.7 weight % of the biologically active complex for the invention.

In the seventh aspect, the invention relates to a cleanser for surfaces, contacting hand skin, containing 0.3-0.7 weight % of the biologically active complex for the invention.

In the eighth aspect, the invention relates to the non-therapeutic cosmetic use of the biologically active complex for to the invention to regulate the skin microbiota, including inhibition of the growth of the population of opportunistic microorganisms, including bacteria, fungi, maintaining a stable state of the resident microflora.

### DETAILED SPECIFICATION

The invention is set out in the appended set of claims. The present invention can be used to manufacture cosmetic products for hand and/or body care, as well as household cleansers.

Within the invention is the biologically active complex is designed for use (the biologically active complex for use) in cosmetic products and/or household cleansers. The biologically active complex can be used in perfume and/cosmetic products, agents, compositions, formulations (cosmetic products). The biologically active complex can be used in products, agents, compositions, household cleansers.

The biologically active complex for the invention contains or comprises:
a) tea tree essential oil, such as *Melaleuca alternifolia,*
b) alpha-bisabolol,
c) 1,8-cineol, derived from eucalyptus leaves Eucalyptus spp.,
d) aqueous solution of citric acid and silver citrate.

**In** the complex for the invention, the specified aqueous solution contains 19-23 weight % citric acids and 2280-2640 parts per million of silver citrate.

The specified aqueous solution may contain nearly 19, 19.5, 20, 20.5, 21, 21.5, 22, 22.5 or 23 weight % of citric acid, or 19, 19.5, 20, 20.5, 21, 21.5, 22, 22.5 or 23 weight % of citric acid from the mass of the complex for the invention.

The specified aqueous solution may contain nearly 2280, 2300, 2350, 2400, 2450, 2500, 2550, 2600 or 2640 parts per million of silver citrate, or 2280, 2300, 2350, 2400, 2450, 2500, 2550, 2600 or 2640 parts per million of silver citrate.

In the complex for the invention, mass ratio of a:b:c:d components is (0.1-0.5):(0.05-0.3):(0.05-0.35):(0.01-0.2) correspondingly.

In the specified mass ratio, tea tree essential oil may be nearly 0.1, 0.2, 0.3, 0.4 or 0.5, or 0.1, 0.2, 0.3, 0.4 or 0.5. Tea tree essential oil can be weight % nearly 0.1, 0.2, 0.3, 0.4 or 0.5, or, in weight %: 0.1, 0.2, 0.3, 0.4 or 0.5 of the mass of the complex for the invention.

In the specified mass ratio, alpha-bisabolol may be nearly 0.05, 0.1, 0.15, 0.2, 0.25 or 0.3, or 0.05, 0.1, 0.15, 0.2, 0.25 or 0.3. Alpha-bisabolol may be nearly, weight %, 0.05, 0.1, 0.15, 0.2, 0.25 or, in weight %, 0.3, or 0.05, 0.1, 0.15, 0.2, 0.25 or 0.3 of the mass of the complex for the invention.

In the specified mass ratio, 1,8-cineol may be nearly 0.05, 0.1, 0.15, 0.2, 0.25, 0.3 or 0.35, or 0.05, 0.1, 0.15, 0.2, 0.25, 0.3 or 0.35. 1,8-cineol may be nearly, weight %, 0.05, 0.1, 0.15, 0.2, 0.25, 0.3 or 0.35 or, in weight %, 0.05, 0.1, 0.15, 0.2, 0.25, 0.3 or 0.35 of the mass of the complex for the invention.

In the specified mass ratio, aqueous solution of citric acid and silver citrate may be nearly 0.01, 0.02, 0.03, 0.04, 0.05, 0.06, 0.07, 0.08, 0.09 or 0.1, or 0.01, 0.02, 0.03, 0.04, 0.05, 0.06, 0.07, 0.08, 0.09, 0.1, 0.15 or 0.2. Aqueous solution of citric acid and silver citrate may be nearly, weight %, 0.1, or 0.01, 0.02, 0.03, 0.04, 0.05, 0.06, 0.07, 0.08, 0.09, 0.1, 0.15 or 0.2, or, in weight %, 0.1, or 0.01, 0.02, 0.03, 0.04, 0.05, 0.06, 0.07, 0.08, 0.09, 0.1, 0.15 or 0.2 of the mass of the complex for the invention.

A person skilled in the art understands that the technical result is achieved not only with the exact value of the quantitative parameter, but also in the proximity to it, within the measurement error of such a parameter associated with various factors. In this regard, in the framework of this invention, when indicating "nearly" with any value of the parameter, it is understood that such a measurement error or interpretation error is taken into account with a certain accuracy of ± 10% of the specified value of the quantitative parameter. In other words, if the term "nearly 5" is used, then it should be interpreted as clear and unambiguously understood by a specialist both on the basis of the prior art and within the framework of this invention, relating to a certain numerical characteristic, and covering values that are in the range of values "5±10%", i.e., the interval "from 4.5 to 5.5", including boundary values, is meant.

Eucalyptus species suggested for use include *E. globulus, E. camaldulensis, E. grandis, E. loxophleba, E. leocoxylon, E. smithii, E. maideni, E. bicostata, E. cineria* and others that accept 1,8-cineol not less than 70% (v/v).

The biologically active complex preferably does not contain other active ingredients and/or adjuvants, such as cosmetic active agents and/or cosmetically acceptable adjuvants, but still may contain them. Such substances may be, or are agents conventionally used in the art and well known to those skilled in the art. The addition of these agents to the composition of the complex according to the invention does not cancel the achievement of the claimed technical results, but can improve them.

As defined in the claims, the biologically active complex is designed for use in cosmetic products, e.g. cosmetic products as liquid soap, shower gel, scalp care product, e.g. shampoo, hair conditioner, etc., body skin care product, e.g. soap liquid or solid bar soap, body gel (shower gel), intimate hygiene products, etc., cosmetic hygiene product that does not contain alcohol (ethyl, isopropyl, etc.) alcohol. Such product may contain about 0.3-0.7 weight % of the biologically active complex of the invention, e.g., about 0.3, 0.4, 0.5, 0.6 or 0.7 weight %, or 0.3, 0.4, 0.5, 0.6 or 0.7 weight % of the product weight. If this is required by the formulation being developed for a particular cosmetic product, such as, including, but not limited to, scalp care products, such product may contain an increased amount of the complex according to the invention, for example, up to 2 weight % of the weight of the product which can be determined by a skilled person. In this case, the product may contain about 0.3, 0.4, 0.5, 0.6, 0.7, 0.8, 0.9, 1, 1.5, or 2 weight%, or 0.3, 0.4, 0.5, 0.6, 0.7, 0.8, 0.9, 1, 1.5 or 2weight % of the weight of the product.

As defined in the claims, the biologically active complex is designed for use in household cleansers. Such product may contain about 0.3-0.7 weight % of the biologically active complex for the invention, e.g., nearly 0.3, 0.4, 0.5, 0.6 or 0.7 weight %, or 0.3, 0 .4, 0.5, 0.6 or 0.7 weight % of the product weight. If this is required by the formulation being developed for a particular household cleanser, such product may contain an increased amount of the complex of the invention, for example, up to 2 weight % of the weight of the product, which can be determined by a skilled person. In this case, the product may contain about 0.3, 0.4, 0.5, 0.6, 0.7, 0.8, 0.9, 1, 1.5, or 2 weight%, or 0.3, 0.4, 0.5, 0.6, 0.7, 0.8, 0.9, 1, 1.5 or 2weight % of the weight of the product.

The most preferred, but not limiting embodiment of the biologically active complex is a complex consisting of:
a) tea tree essential oil (*Melaleuca alternifolia*),
b) alpha-bisabolol,
c) 1,8-cineol, derived from eucalyptus leaves Eucalyptus spp.,
d) aqueous solution of citric acid and silver citrate,
where the specified aqueous solution contains 19-23 weight % of citric acid and 2280-2640 parts per million of silver citrate; and where mass ratio of a:b:c:d components makes (0.1-0.5):(0.05-0.3):(0.05-0.35):(0.01-0.2), correspondingly.

As defined in claim 10, the biologically active complex under any of claims 1-4 is designed to regulate the skin microbiota, including inhibition of the growth of the population of opportunistic microorganisms, including bacteria, fungi, maintaining a stable state of the resident microflora, a pronounced antibacterial effect on the transient microflora, the destruction of biofilms of opportunistic pathogens.

All mass fractions, mass parts, mass percentages, as well as volume fractions, volume parts, volume percentages in the present disclosure are given in relation to the preparation, agent, composition or product to which they refer in the indicated context.

The biologically active complex is different in that tea tree essential oil is a commercially available product or a custom-synthesized (extracted) product and is a substance with Registration Number CAS 68647-73-4.

The biologically active complex is different in that alpha-bisabolol is a commercially available product or a synthesized (extracted) product under an order and is a substance with Registration Number CAS 515-69-5/23089-26-1.

The biologically active complex is different in that 1,8-cineol is a commercially available product or a synthesized (extracted) product under an order and is a substance with Registration Number CAS 470-82-6.

The biologically active complex is different in that aqueous solution of citric acid and silver citrate is a commercially available product or a synthesized (extracted) product under an order and is a substance with Registration Number CAS 126-45-4.

In the products of the invention, cosmetically acceptable adjuvants may be selected from (INCI), but not limited to: Aqua, Sodium Coco-Sulfate, Sunfloweroyl Methylglucamide, Coco-Glucoside, Glycerin, D-panthenol, Cocamidopropyl Betaine, Lactic Acid, Citric acid, Benzyl Alcohol, Phenoxyethanol, Glyceryl Oleate, Parfum, Sodium Chloride, Tetrasodium Glutamate Diacetate, Benzoic Acid, and/or Dehydroacetic Acid, all of which are commercially available.

The composition for the invention may contain, weight %:

| | |
|---|---|
| Tea tree essential oil *Melaleuca alternifolia leaf oil* | 0.10-0.50, |
| Alpha-bisabolol *Bisabolol rac.* | 0.05-0.30, |
| 1,8-cineol *Eucalyptol* | 0.05-0.35, |
| Aqueous solution of citric acid and silver citrate *(Aqua, Citric acid and Silver citrate)* | 0.01-0.20. |

The present invention can be used to manufacture cosmetic products for skin care and refers to the biologically active complex, containing the highly efficient components: a) tea tree essential oil *Melaleuca alternifolia (Tea tree) oil,* b) alpha-bisabolol, derived from oils or synthetically, c) 1,8-cineol, derived from eucalyptus leaves Eucalyptus spp., d) aqueous solution of citric acid and silver citrate, to maintain skin hygiene.

Tea tree essential oil *Melaleuca alternifolia (tea tree) oil* (INCI: Melaleuca alternifolia leaf oil) is a commercially available ingredient of cosmetic products, well known by persons skilled in the art.

Alpha-bisabolol (INCI: Bisabolol rac.) is a commercially available ingredient of cosmetic products, well known by persons skilled in the art.

1,8-cineol (INCI: Eucalyptol) is a commercially available ingredient of cosmetic products, well known by persons skilled in the art.

Aqueous solution of citric acid and silver citrate (INCI: Aqua (and) Citric acid (and) Silver citrate) is a commercially available ingredient of cosmetic products, well known by persons skilled in the art.

In the preparation for the invention, acceptable adjuvants may be selected from the list, including but not limited to: Aqua, Sodium Coco-Sulfate, Sodium Laureth Sulphate, Decyl glucoside, Sunfloweroyl Methylglucamide, Coco-Glucoside, Cocamidopropyl Betaine, Alkylpolyglycoside C10-16, Alkyl polyglycoside C8-10, Caprylyl & Decyl Glucoside, D-Glucopyranose, oligomeric, C10-16-alkyl glycosides, D-Glucopyranose, oligomers, decyl octyl glycosides, Polyalkylglycoside C8-16, Glyceryl Oleate, Glyceryl Stearate, Sodium Cocoamphoacetate, Sodium Myristoyl Sarcosinate, Sodium Lauryl Sarcosinate, Sodium C12-C18 alkyl sulfate, Potassium cocoate, Glycereth-6 Cocoate, Sodium Cocoyl Glutamate, Sodium 2-Ethylhexyl Iminodipropionate, Laureth-9 & Laureth-3, Polyethylene Glycol (7) Lauryl Ether, Lauryl glucoside, Polyglyceryl-6 Caprylate (and) Polyglyceryl-3 Cocoate (and) Polyglyceryl-4 Caprate (and) Polyglyceryl-6 Ricinoleate, Polyglyceryl-4 Laurate/Sebacate (and) Polyglyceryl-6 Caprylate/Caprate, Sodium Carboxymethyl Carbohydrate, Glycerin, D-panthenol, Xanthan gum, Gossypium herbaceum seed extract, Lactic Acid, Citric Acid, Glycolic Acid, Sodium hydroxide, Trisodium citrate dehydrate, Sodium sulphate, Sodium carbonate, Sodium bicarbonate, Benzyl Alcohol, Phenoxyethanol, Benzoic Acid, Dehydroacetic Acid, Potassium sorbate, Sodium benzoate, Methylisothiazolinone, Benzoisothiazolinone, Sodium Chloride, Tetrasodium Glutamate Diacetate, Trisodium salt of Methylglycinediacetic acid, Parfum, Eucalyptus essential oil, Citrus paradisi (Grapefruit) essential oil, Melissa officinalis (Melissa) oil, Mentha piperita (Pippermint) essential oil, Lavandula angustifolia (Lavender) essential oil, Citrus Nobilis (Tangerine) essential oil or Propionic Acid Bacteria Lysate, all of which are commercially available.

### EXPERIMENTAL

The following examples are provided.

Studies have been conducted to evaluate the effectiveness of the composition for the invention.

### Example 1.

A study of antibacterial activity of liquid hand soap with the components of the antibacterial biologically active quad complex was made, in particular, tea tree essential oil *Melaleuca alternifolia (Tea tree) leaf oil,* alpha-bisabolol, 1,8-cineol and aqueous solution of citric acid and silver citrate.

A mixture, containing Aqua, Sodium Coco-Sulfate, Coco-Glucoside, Glycerin, Cocamidopropyl Betaine, Lactic Acid, Benzyl Alcohol, Tetrasodium Glutamate Diacetate, Benzoic Acid, as per Table No. 1, was used as the base to introduce the components of the complex.

The base of liquid soap for hand skin care was made as follows: a solubilizer, glycerin, and a chelating agent, glutamate diacetate tetrasodium salt were mixed with purified water, while stirring in an ordinary mixer until the mixture was homogeneous. Next, the surfactants, sodium coco-sulfate, coco glucoside, cocamidopropylbetaine were added. At the end, organic acid was added to adjust pH, as well as preservatives, benzyl alcohol, and benzoic acid to maintain microbiological stability throughout the shelf life. The ingredients were mixed until a homogeneous clear solution was obtained.

**Table No. 1. Composition of the liquid soap base**

| Item No. | Component |
|---|---|
| 1 | Purified water |
| 2 | Glycerin 99,5% |
| 3 | Sodium Coco-Sulfate |
| 4 | Coco glucoside |
| 5 | Cocamidopropylbetaine |
| 6 | Glutamate diacetate tetrasodium salt |
| 7 | Benzyl alcohol and benzoic acid |

The method of analysis is based on double dilution technique and consists in preparing a suspension of *Escherichia coli* and *Staphylococcus aureus* bacterial cells separately at a concentration of 1x10⁹ cells/ml. Next, 0.2 ml of a suspension of *Escherichia coli* or *Staphylococcus aureus* bacterial cells is added to 1.8 ml of a 5% liquid soap solution containing components of the biologically active quad complex. In 30 minutes, 1 ml of the resulting mixture is added to a test tube with 9 ml of sterile saline 0.9%. In 15 more minutes, a series of dilutions is prepared in sterile saline to a concentration of 10³ cells/ml. 0.1 ml of a solution of the initial concentration and successive dilutions is added to the surface of cups with a special nutrient medium for the growth of *Escherichia coli* and *Staphylococcus aureus* separately. The cultures are incubated for 48 hours at 37 degrees Celsius, and the colonies are counted with a standard method.

The parameter for the overall assessment of the antibacterial activity of the biologically active quad complex in the composition of liquid soap was the count of the number of grown colonies (CFU/ml), determined by the microbiologist based on the results of the study. The indicator of the number of colony-forming units of bacteria CFU/ml allows evaluating the severity of antibacterial activity against the selected strain of *Escherichia coli* or *Staphylococcus aureus* bacteria with daily use of the biologically active quad complex as part of liquid soap. The general positive trend in the use of liquid soap with the claimed complex is a decrease in the biodiversity of pathogenic strains of *Escherichia coli* and *Staphylococcus aureus* on the surface of the skin of the hands, which will stimulate the growth of beneficial microflora to maintain hygiene and dermatological comfort.

Following the results of the assessment of antibacterial activity, the tested biologically active quad complex in the composition of liquid soap has a pronounced antibacterial effect against *Escherichia coli* and *Staphylococcus aureus* compared to the control. The control version is a suspension of *Escherichia coli* or *Staphylococcus aureus* bacterial cells in 1.8 ml of a 5% liquid soap solution that does not contain components of the biologically active quad complex.

At the end of the study, pronounced changes in the estimated indicator of antibacterial activity against *Escherichia coli* were observed. According to the dynamics of the indicator, relative to the initial concentration of *E. coli,* the concentration of bacteria decreased by 99.97% at average when studying various dilutions, which is a coefficient of reduction in contamination of 4log₁₀. The control version also showed a decrease in the number of pathogenic bacteria *E. coli,* but by a smaller amount.

To compare the results of the control and experimental groups, statistical processing of the results was performed. To compare two samples with a known distribution law, a parametric Student's t-test was used for two independent samples. This method allows testing the hypothesis that the average values of two general populations, from which two compared independent samples are extracted, differ from each other.

The t-test (practical) and t-test (0.95,f) values are 8.04 and 4.31, correspondingly. Thus, between sample 1 (control version) and sample 2 (experimental version) there are statistically significant differences in the concentration of *E. coli* with an error probability below 5%. According to the results of statistical data processing, the biologically active quad complex in the composition of liquid soap has significant antibacterial activity compared to the control sample against *Escherichia coli* as a representative of the pathogenic gram-negative microflora of the hand skin (Table No. 2).

**Table No. 2. Antibacterial activity estimation against E. coli**

| Concentration E.coli, c/ml | Tested samples | | Dynamics of the indicator, in % |
|---|---|---|---|
| | Control version | Experimental version | |
| 10⁷ | Confluent growth | 1000 CFU | 99.99% |
| 10⁶ | Confluent growth | 439 CFU | 99.96% |
| 10⁵ | 1330 CFU | 83 CFU | 99.92% |
| 10⁴ | 329 CFU | No growth | 100% |
| 10³ | 31 CFU | No growth | 100% |
| Average value: | | | **99.97%** |

Moreover, at the end of the study, pronounced changes in the estimated indicator of antibacterial activity against *Staphylococcus aureus* were observed. According to the dynamics of the indicator, relative to the initial concentration of *S. aureus,* the concentration of bacteria decreased by 99.25% on average when studying various dilutions, which is a coefficient of reduction in contamination of 3log₁₀. The control version also showed a decrease in the number of pathogenic bacteria *S. aureus,* but by a smaller amount.

To compare the results of the control and experimental groups, statistical processing of the results was performed. To compare two samples with a known distribution law, a parametric Student's t-test was used for two independent samples. This method allows testing the hypothesis that the average values of two general populations, from which two compared independent samples are extracted, differ from each other.

The t-test (practical) and t-test (0.95,f) values are 14.09 and 4.31, correspondingly. As - test (practical) > t-test (0.95,f). Thus, between sample 1 (control version) and sample 2 (experimental version) there are statistically significant differences in the concentration of S. *aureus* with an error probability below 5%. According to the results of statistical data processing, the biologically active quad complex in the composition of liquid soap has significant antibacterial activity compared to the control sample against *Staphylococcus aureus* as a representative of the pathogenic gram-positive microflora of the hand skin, which is able to form stable biofilms on various surfaces (Table No. 3).

**Table No. 3. Antibacterial activity estimation against S. aureus**

| Concentration of S. aureus, c/ml | Tested samples | | Tested samples |
|---|---|---|---|
| | Control version | Control version | |
| 10⁶ | Confluent growth | 1010 CFU | 99.90% |
| 10⁵ | 2155 CFU | 523 CFU | 99.48% |
| 10⁴ | 201 CFU | 108 CFU | 98.92% |
| 10³ | 16 CFU | 13 CFU | 98.70% |
| Average value: | | | **99.25%** |

A microbiota is a harmonious system, so it is important to maintain a balance of microorganisms both on skin and on surfaces. In particular, *E. coli* and *Staphylococcus aureus,* together with other opportunistic microorganisms, are able to form stable biofilms from the exopolysaccharide matrix and contribute to the development of various diseases due to the release of toxins [E. Scott, S. Bloomfield. The survival and transfer of microbial contamination via cloths, hands and utensils. J. Appl. Bacteriol. 68 (1990) 271-278].

The liquid soap base of the composition of will gently cleanse hand skin impurities and increase the access of biologically active substances (BAS) of the quad complex to hardly accessible areas of the skin, thereby increasing the effectiveness of BAS against opportunistic microflora. The study of liquid soap with the claimed biologically active quad complex led to a high antibacterial effect against opportunistic *Escherichia coli* and *Staphylococcus aureus,* which are a causative factor in the development of dermatological and gastrointestinal diseases and a representative of the transient microflora of hand skin. Reducing the alpha and beta biodiversity of opportunistic strains, in particular *Escherichia coli* and *Staphylococcus aureus,* will help shift alpha-biodiversity towards the beneficial resident microflora of the skin of the hands and improve the dermatological condition of skin in general.

Since the composition contains an aqueous solution of citric acid and silver citrate containing active Ag + silver ions, and sesquiterpene alcohol alpha-bisabolol with a synergetic antibacterial effect due to the suppression of unformed biofilms of transient pathogenic skin bacteria, the amount of opportunistic pathogenic microorganisms in the upper layers of the skin will decrease with prolonged use. In joint use, the aqueous solution of citric acid and silver citrate as a carrier of Ag + silver ions and alpha-bisabolol inhibit the enzyme systems of microorganisms that provide metabolism and cell division. When combined with penetration enhancers such as 1,8-cineol, the components have a synergetic antibacterial effect by improving the permeability of the components through the lipophilic membranes of bacteria and the formation of liposomal structures in conjunction with tea tree essential oil. The components of tea tree essential oil block the action of respiratory enzymes and bind K + potassium ions, which cause osmotic shock and lysis of bacterial cells.

Thus, the combination of the components makes it possible to achieve a synergetic effect against opportunistic microorganisms due to the destruction of biofilms of pathogenic microorganisms and a minimal risk of developing resistance in bacteria, which allows it to be regularly used as part of various cosmetic products, as well as household cleansers.

### Example 2.

A study of antibacterial activity of liquid hand soap with the components of the antibacterial biologically active quad complex was made, in particular, tea tree essential oil *Melaleuca alternifolia (Tea tree) leaf oil,* alpha-bisabolol, 1,8-cineol and aqueous solution of citric acid and silver citrate.

A mixture, specified in Table No.1, was used as the base to introduce the components of the complex. The base of the liquid soap was made with the method, described in Example 1.

The analysis technique is based on Procedure Details MUK 4.2.801-99 "Methods of Microbiological Control of Perfumery and Cosmetic Products", double dilution technique. 5 pathogenic test microorganisms were selected, widely represented on hand skin and responsible for various bacterial diseases: *Staphylococcus aureus* ATCC 6538-P, *Escherichia coli* ATCC 25922, *Bacillus cereus* ATCC 10702, *Pseudomonas aeruginosa* ATCC 9027, *Candida albicans* ATCC 10231. The following diagnostic media were used for growth assessment: buffer solution for *Bacillus cereus* and *Candida albicans,* McConkey broth for *Escherichia coli,* saline broth for *Staphylococcus aureus,* meat peptone broth with glucose for *Pseudomonas aeruginosa.*

The dilution method considers taking a suspension of bacterial cells of test strains in a certain concentration as the control. At the same time, a liquid soap sample is diluted 1: 1000 with the addition of a suspension of test microorganisms. Next, the solution of the initial concentration and the dilution solution are applied to the surface of the cups with a special nutrient medium for the growth of microorganisms. The cultures are incubated for 48 hours at a temperature of 37 degrees Celsius and growth is determined by a visual method.

The parameter for the overall assessment of the antibacterial activity of the biologically active quad complex in the composition of liquid soap was the presence or no growth of test microorganisms, determined by the microbiologist based on the test result. The bacterial growth rate allows evaluating the instant antibacterial effect against the selected test strain of microorganisms with daily use of the biologically active quad complex in liquid soap. The general positive trend in the use of liquid soap with the claimed complex is no growth of opportunistic microorganisms on the surface of the skin, which will stimulate the growth of beneficial microflora to maintain hygiene and dermatological comfort.

According to the results of the assessment of antibacterial activity, the tested biologically active quad complex in the composition of liquid soap has a pronounced antibacterial effect against 5 test microorganisms compared to the control. The control version is a suspension of bacterial cells of test microorganisms in a liquid soap solution that does not contain components of the biologically active quad complex.

At the end of the study, pronounced changes in the estimated indicator, namely antibacterial activity, were observed. According to the dynamics of the indicator, relative to the initial concentration of test strains (*Staphylococcus aureus, Escherichia coli, Pseudomonas aeruginosa, Bacillus cereus, Candida albicans*), the concentration of all test microorganisms decreased by 99.90% on average when studying various dilutions, which is a reduction factor of 3log₁₀. The control version demonstrated a growth of test microorganisms, i.e. lack of antibacterial activity (Table No. 4).

**Table No. 4. Antibacterial activity estimation against 5 test-strains**

| Test-microorganism | Tested samples | |
|---|---|---|
| | Control version (culture control) | Experimental version (1:1000) |
| Bacillus cereus ATCC 10702 | + | - |
| Escherichia coli ATCC 25992 | + | - |
| Pseudomonas aeruginosa ATCC 9027 | + | - |
| Staphylococcus aureus ATCC 6538-P | + | - |
| Candida albicans ATCC 10231 | + | - |
| References: | | |
| «+» - confluent growth-of test microorganisms as in the control; | | |
| «±» - retarded or depressed growth; | | |
| «-» - no growth. | | |

A microbiota is a harmonious system, so it is important to maintain a balance of microorganisms both on skin and on surfaces. In particular, opportunistic resistant *Staphylococcus aureus, Escherichia coli* and *Pseudomonas aeruginosa,* together with other opportunistic microorganisms such as *Candida albicans* and *Bacillus cereus,* can form stable biofilms from the exopolysaccharide matrix and contribute to the development of various dermatological and systemic diseases. The peculiarity of these microorganisms is that they are often multi-resistant, as they have developed drug resistance to many antibacterial substances, which complicates their growth inhibition and death in the current epidemiological situation.

The base of the liquid soap composition will gently cleanse hand skin impurities and increase the access of biologically active substances (BAS) of the quad complex to hardly accessible areas of the skin, thereby increasing the effectiveness of BAS against opportunistic microflora. The study of liquid soap with the claimed biologically active quad complex has led to a high antibacterial effect against opportunistic strains that are a causative factor in the development of dermatological and systemic diseases and representatives of the transient microflora of the skin of the hands. Reducing the biodiversity of opportunistic strains, in particular those forming stable biofilms, will help shift alpha and beta biodiversity towards beneficial resident skin microflora and improve the dermatological condition of the skin in general.

Since the composition contains an aqueous solution of citric acid and silver citrate containing active Ag + silver ions, and alpha-bisabolol sesquiterpene alcohol with a synergetic antibacterial effect due to the suppression of unformed biofilms of transient pathogenic skin bacteria, the number of pathogenic microorganisms in the upper layers of the skin will decrease with prolonged use. At the same time, silver citrate as a carrier of Ag + silver ions and alpha-bisabolol inhibit the enzyme systems of microorganisms that provide metabolism and cell division. In joint use with penetration enhancers such as 1,8-cineol, the components have a synergetic antibacterial effect by improving the permeability of the components through the lipophilic membranes of bacteria and the formation of liposomal structures in conjunction with tea tree essential oil. The components of tea tree essential oil block the action of respiratory enzymes and bind K + potassium ions, which cause osmotic shock and lysis of bacterial cells.

Thus, the combination of components makes it possible to achieve a synergetic effect against opportunistic microorganisms due to the destruction of biofilms of pathogenic microorganisms and a minimal risk of developing resistance in bacteria, which allows it to be regularly used as part of various cosmetic products, as well as household cleansers.

### Example 3.

A study of antibacterial activity of samples with the components of the antibacterial biologically active quad complex was made, in particular, tea tree essential oil *Melaleuca alternifolia (Tea tree) leaf oil,* alpha-bisabolol, 1,8-cineol and aqueous solution of citric acid and silver citrate.

A mixture consisting of purified water and 0.5% Tween 80 was used as a base to include the components of the complex. Samples were prepared using a magnetic stirrer by solubilizing the components of the quad complex in Tween 80 and gradually introducing them into purified water at 350 rpm. The stability of the samples was evaluated visually, by the absence of separation of the fatty phase from the aqueous solution.

2 strains of microorganisms of normal microflora, widely represented on the skin of the hands, were selected: *Staphylococcus epidermidis* and *Micrococcus luteus.* Microorganisms of transient opportunistic microflora present on the skin of the hands were also selected: *Staphylococcus aureus, Escherichia coli* and fungi of *Candida albicans* genus. Microorganisms were obtained by washing from the skin of the hands of subjects or using standard strains, cultivating isolated microorganisms and counting the number of grown colonies of microorganisms.

The method of the analysis is based on the double dilution method and consists in preparing a suspension of bacterial cells of selected microorganisms (S. *aureus, E. coli, C. albicans, S. epidermidis, M. luteus*) separately at a concentration of 1x10⁹ cells/ml. Next, 0.2 ml of a suspension of bacterial cells is added to 1.8 ml of a 5% solution of antibacterial components. In 30 minutes, 1 ml of the resulting mixture is added to a test tube with 9 ml of sterile saline 0.9%. In 15 more minutes, a series of dilutions is prepared in sterile saline to a concentration of 103 cells/ml. 0.1 ml of a solution of the initial concentration and successive dilutions is added to the surface of cups with a special nutrient medium for the growth of selected microorganisms separately. The cultures are incubated for 48 hours at 37 degrees Celsius and the colonies are counted by the standard method.

The parameter for the overall assessment of the effect of the biologically active quad complex on the growth of representatives of the normal microflora was the presence or no growth of microorganisms, determined by the microbiologist based on the results of the study. The indicator of bacterial growth allows evaluating the effect of microflora on microorganisms and drawing a conclusion about the effect of the quad complex on the microflora of hand skin. The general positive trend in the use of the components of the claimed quad complex is the absence of a negative effect on the normal microflora of the skin of the hands and a pronounced antibacterial effect on the transient microflora, which will ensure the maintenance of natural non-pathogenic microflora for dermatological comfort and hygiene of hand skin.

Based on the results of assessing the influence on the growth of representatives of normal and transient microflora, the tested biologically active quad complex and its components do not have an antibacterial effect on representatives of the normoflora of hand skin. At the end of the study, the growth of bacteria strains of *Staphylococcus epidermidis* and *Micrococcus luteus* was observed. However, the components of the complex differently affect the transient opportunistic human microflora, having a bacteriostatic effect on the growth of *Staphylococcus aureus, Escherichia coli* and *Candida albicans.* Individually, the components are able to inhibit the growth of opportunistic microorganisms or inhibit growth in most culture areas, however, when combined, a synergistic antibacterial effect can be achieved for the predominant absence of microorganism growth (Table No. 5).

**Table No. 5. Effect of antibacterial basic samples on the growth of representatives of hand skin microflora**

| Experimental samples | Test-strains of microorganisms | | | | |
|---|---|---|---|---|---|
| | S. epidermidis | M.luteus | S. aureus | E. coli | C. albicans |
| Tea tree essential oil 0,30% | - | - | ± | + | ± |
| Alpha-bisabolol 0,10% | - | - | ± | ± | - |
| 1,8-cineol 0,15% | - | - | - | ± | - |
| Aqueous solution of citric acid and silver citrate 0,01% | - | - | - | ± | - |
| Biologically active quad complex | - | - | ++ | ++ | + |
| Ethyl alcohol 96% | ++ | ++ | ++ | ++ | ++ |
| Hydrogen peroxide 3% | + | + | + | + | + |
| Benzalkonium chloride 0,1% | ++ | ++ | ++ | ++ | ++ |
| Purified water | - | - | - | - | - |
| References: | | | | | |
| «++» - no growth; | | | | | |
| «+» - depressed growth in most areas; | | | | | |
| «±» - retarded growth in some areas; | | | | | |
| «-» - confluent growth-of test microorganisms. | | | | | |

A microbiota is a harmonious system, so it is important to maintain a balance of microorganisms both on skin and on surfaces. In particular, *Staphylococcus aureus,* together with other opportunistic microorganisms, such as *Escherichia coli,* are able to form stable biofilms from the exopolysaccharide matrix and contribute to the development of various diseases such as atopic dermatitis [E. Scott, S. Bloomfield. The survival and transfer of microbial contamination via cloths, hands and utensils. J. Appl. Bacteriol. 68 (1990) 271-278].

The components of the biologically active quad complex specifically affect the growth of opportunistic microorganisms, to which resistance quickly develops and which form stable protective biofilms, but do not have a negative effect on resident microorganisms, which allows providing targeted protection against the development of opportunistic representatives and maintaining harmonious biodiversity microflora. A decrease in the biodiversity of pathogenic strains will contribute to a shift in alpha and beta biodiversity towards the beneficial resident microflora of the skin of the hands and an improvement in the dermatological condition of the skin in general.

Since the composition contains an aqueous solution of citric acid and silver citrate containing active Ag + silver ions, and alpha-bisabolol sesquiterpene alcohol with a synergetic antibacterial effect due to the suppression of unformed biofilms of transient pathogenic skin bacteria, the number of pathogenic microorganisms in the upper layers of the skin will decrease with prolonged use. In joint use with penetration enhancers such as 1,8-cineol, the components have a synergetic antibacterial effect by improving the permeability of the components through the lipophilic membranes of bacteria and the formation of liposomal structures in conjunction with tea tree essential oil. The components of tea tree essential oil block the action of respiratory enzymes and bind K + potassium ions, which cause osmotic shock and lysis of bacterial cells. It should be noted that the components of the complex, individually and together, do not have a bacteriostatic effect on the representatives of the resident microflora of the skin of the hands, which maintains the biodiversity of normal microflora and provides natural skin protection.

Thus, combining the components into a single complex makes it possible to achieve a synergetic effect against opportunistic microorganisms (*S. aureus, E. coli, C. albicans*) and the preservation of representatives of the normal microflora (*S. epidermidis, M. luteus*), capable of providing natural protection. from overgrowth of transient flora. The claimed biologically active quad complex has a high antibacterial effect against opportunistic transient strains, which are a causative factor in the development of dermatological and systemic diseases and representatives of the transient microflora of the skin of the hands. A decrease in the representation of opportunistic strains, in particular those forming stable biofilms, will contribute to the shift of alpha and beta biodiversity towards the beneficial resident microflora of hand skin and improve the dermatological condition of the skin in general. Since the normal microflora of the skin is preserved at the site of application, it is possible to use it as part of various cosmetic products and household cleansers on a regular basis.

### Example 4.

A clinical laboratory study was conducted with the participation of healthy subjects to evaluate the effect of the antibacterial biologically active quad complex, namely tea tree essential oil *Melaleuca alternifolia (Tea tree) leaf oil,* alpha-bisabolol, 1,8-cineol and an aqueous solution of citric acid and silver citrate, on the microflora of the skin with a single and regular use of liquid soap as a basic cosmetic product.

A mixture, containing the following components: Aqua, Sodium Coco-Sulfate, Coco-Glucoside, Glycerin, Cocamidopropyl Betaine, Lactic Acid, Benzyl Alcohol, Tetrasodium Glutamate Diacetate, Benzoic Acid, as per Table No.1, was used as the basis to introduce the components of the complex. The preparation process for the liquid soap was described in Example 1.

To determine the effect of the biologically active quad complex in liquid soap on the skin microflora, an open prospective double-blind comparative study was conducted on 40 subjects. During the trial, the subjects were divided into 2 groups: the experimental group of 20 subjects who used liquid soap with the antibacterial bioactive quad complex, and the control group who used liquid soap without the antibacterial bioactive quad complex. All subjects included in the study washed their hands in warm running water for at least 30 seconds using 1.5 ml of liquid soap, actively lathering the skin of their hands until abundant foam appeared. The subjects of the both groups used the product in accordance with the recommendations for use, at least 4 times a day. The total duration of the study for the subjects was 21 days, with interim visits on days 1 and 7 of the study.

For microbiological research, material was taken from the skin surface of the hands using a sterile swab, wiping the skin of the palms for 30 seconds. After that, the swabs were immersed in a sterile transport nutrient medium (Stuart's medium) and delivered to the microbiological laboratory for analysis within an hour. Inoculations of the material from the liquid transport medium were carried out on differential diagnostic dense nutrient media.

The parameter for the overall assessment of the impact on the microflora of the skin of the hands was a change in the biodiversity of the microflora of the skin of the hands (in quantitative units), namely the number of grown microorganisms obtained from washings from the skin of the hands of subjects. A general positive trend is a decrease in the alpha and beta biodiversity of microflora on hand skin, with a predominant inhibition of opportunistic microorganisms.

For quantitative data, the group arithmetic mean (M) and standard deviation (SD) were calculated. The obtained data were processed using MS Excel software. The probability of differences in mean indicators at different points in time was determined using Student's t-test with a normal distribution in independent and dependent samples. The differences were considered significant at a significance level of p<0.05.

The results of the bacteriological study of clinical material obtained from the skin of the hands of subjects (Table No.6) demonstrated a significant decrease in microbial contamination in the main group compared to the control (p<0.05) with long-term use of a cosmetic product. Despite the fact that healthy subjects with healthy hand skin, without visible damage, participated in the tests, according to the results of comparing the dynamics of the indicator, it was found that during the use of liquid soap with the antibacterial biologically active quad complex, a statistically significant change in the indicator was observed (p<0.05 , t-Student for 2 dependent samples). Prior to the use of liquid soap with antibacterial components, mainly S. epidermidis, S. haemolyticus, Micrococcus spp., other gram-positive rods, as well as colonies of opportunistic microorganisms were sown. 3 hours after washing the skin of the hands, a sharp decrease in the number of grown colonies was observed due to the washing action of surfactants in the composition of liquid soap and antibacterial components that are part of the biologically active quad complex. Further use of the composition of the soap showed a significant decrease in the number of colonies compared with the first day. The presence of a stable small number of colonies that grew on the 7th and 21st days of the study indicates a pronounced bacteriostatic effect of the biologically active quad complex against opportunistic microorganisms and some representatives of the resident microflora that abundantly inhabit the skin of the hands. Given the fact that the subjects at the baseline showed a good level of microflora and used hygiene products during the COVID-19 pandemic, the observed effect with higher contamination will be more noticeable, and the alpha and beta biodiversity of the microflora is much lower.

In subjects of the control group, who used liquid soap without biologically active quad complex, *S. epidermidis, S. haemolyticus, Micrococcus spp.,* as well as colonies of opportunistic microorganisms were sown before the start of the study. In 3 hours after washing hand skin, a sharp decrease in the number of grown colonies was observed due to the washing action of the surfactant in the composition of the liquid soap. With long-term use, there was a tendency to reduce the sowing of cultures, but less pronounced than in the case of liquid soap with a biologically active quad complex. The presence of a stable significant number of colonies that grew on the 7th and 21st days of the study indicates a very weak bacteriostatic effect of the biologically active quad complex against opportunistic microorganisms and some representatives of the resident microflora that abundantly inhabit hand skin.

**Table No. 6. Results of microbiological tests of skin microflora**

| **Product** | **Parameter** | **Number of grown colonies of microorganisms on dense nutritive media** | | | |
|---|---|---|---|---|---|
| | | **Before using soap** | **In 3 hours after use** | **In 7 days after use** | **In 21 days after use** |
| Liquid soap with the biologically active quad complex | M±SD | 32.30±12.11* | 1.30±2.41 | 6.75±5.87 | 7.00±4.92 |
| | Total CFU in 20 subjects | 646 | 26 | 135 | 140 |
| Liquid soap without the biologically active quad complex | M±SD | 19.70±11.06 | 0.70±1.17 | 15.45±5.98 | 14.35±7.09 |
| | Total CFU in 20 subjects | 394 | 14 | 309 | 287 |

Following the test results, the biologically active quad complex in the composition of liquid soap changes the microflora of the skin of the hands with regular use of the product containing it. The use of liquid soap with the claimed biologically active quad complex led to a pronounced change in the microflora of hand skin, which indicates a decrease in the diversity of transient conditionally pathogenic microorganisms, which are the causative factor of many human diseases in general. The decrease in the biodiversity of opportunistic strains contributed to the shift of beta biodiversity towards the beneficial resident microflora of the skin and the improvement of the dermatological condition of the skin in general, which was noted by all subjects.

Since the biologically active quad complex contains tea tree essential oil (*Melaleuca alternifolia*), alpha-bisabolol, 1,8-cineol and an aqueous solution of citric acid and silver citrate, with a synergetic effect of the components due to the multifactorial action on the compartments of microorganism cells. The base of the composition gently cleansed contamination off hand skin and increased the access of biologically active substances (BAS) of the quad complex to hardly assessable areas of the skin, thereby increasing the effectiveness of BAS against opportunistic microflora. Due to the presence of active Ag + silver ions and sesquiterpene alcohol alpha-bisabolol with a synergistic antibacterial effect due to the suppression of unformed biofilms of transient pathogenic skin bacteria, long-term use will reduce the number of opportunistic microorganisms in the upper layers of the skin. Together, silver citrate as a carrier of Ag + silver ions and alpha-bisabolol inhibit the enzyme systems of microorganisms that provide metabolism and cell division. When used together with the penetration enhancer 1,8-cineol, the components have a synergistic bacteriostatic effect by improving the permeability of the components through the lipophilic membranes of bacteria and the formation of metastable liposomal structures together with tea tree essential oil.

### Example 5.

The method to obtain the composition for to the invention includes the following steps aimed at ensuring high bioavailability of the biologically active substances of the composition. When mixing the components, it is recommended to use the octanol to water coefficient logP_{o/w}, which is a measure of the lipophilicity of substances and determines the affinity for lipophilic structures. Compliance with the rule of mixing components with a decrease in logP will allow the formation of stable liposomal structures, increase the penetration of substances through the membrane of microorganisms and potentiate biological activity for the claimed purposes.

For example, 25 mass parts of tea tree essential oil *Melaleuca alternifolia* with an average logP of 4.13, due to the high content of terpinen-4-ol, γ- and α-terpinene, was taken and mixed with 10 mass parts of alpha-bisabolol with a logP of 3.80 with a magnetic stirrer at 100 rpm. Next, 5 parts of 1,8-cineol were added, derived from eucalyptus leaves Eucalyptus spp., with a logP of 2.82. Next, 1 mass part of aqueous solution of citric acid and silver citrate was added, where the specified aqueous solution contains 19-23 weight % of citric acid and 2280-2640 parts per million of silver citrate, using a homogenizer for effective distribution in the composition. Subsequent inclusion of substances in the composition allows to achieve high stability, potentiates biological activity due to the highest physical and chemical affinity and joint chemically conditioned penetration of the components of the composition.

The prepared composition can be used to manufacture cosmetic products for hand and/or body care, as well as household cleansers. One of the options for implementation is integration into a cosmetic product for washing hands as follows: dissolve surfactants in preheated water purified using a magnetic stirrer. The previously prepared composition is added to glycerin and solubilizer and is stirred until the mixture is homogeneous. A chelating agent and a preservative active at the given pH of the product are then added to maintain microbiological stability over the shelf life additionally provided by this composition. To give a certain flavor, you can include a fragrance or other component.

This product can be used to regulate skin microbiota, including inhibiting the growth of a population of opportunistic microorganisms, including bacteria, fungi, maintaining a stable state of the resident microflora of the skin of the hands, body, scalp, a pronounced antibacterial effect on the transient microflora, destroying biofilms of conditionally pathogenic microorganisms.

Additionally, the composition can be introduced into the following types of products when modifying the base of surfactants: liquid cosmetic soap, liquid cream soap, solid cosmetic soap bars, shower and/or bath gel, bath foam, foam cleanser, anti-dandruff scalp care product, e.g. daily shampoo, balm shampoo, hair and body wash, conditioner shampoo, balm, conditioner, mask, liquid oral hygiene products, e.g. toothpaste, rinse.

Additionally, the composition can be introduced into the following types of products when selecting emulsifying components: cosmetic body skin care product, e.g., hand cream, hand lotion, body cream, body lotion, deodorant, deodorant perfume, cream perfume, solid perfume, cosmetic balm, cosmetic gel.

Additionally, the composition can be used as an infusion for cosmetic wipes intended for hand skin hygiene.

Thus, the achievement of all the expected results was confirmed in the application materials; the high efficiency of the claimed complex and its industrial applicability were demonstrated.

## Claims

1. The biologically active complex designed for use in cosmetic products and/or household cleansers, comprising of:
a) tea tree essential oil,
b) alpha-bisabolol,
c) 1,8-cineol, derived from eucalyptus leaves of *Eucalyptus spp.,*
d) aqueous solution of citric acid and silver citrate,
where the specified aqueous solution contains 19-23 weight % of citric acid and 2280-2640 parts per million of silver citrate; and where mass ratio of a:b:c:d components makes (0.1-0.5):(0.05-0.3):(0.05-0.35):(0.01-0.2), correspondingly.

2. The biologically active complex under Claim 1, wherein said tea tree essential oil is derived from *Melaleuca alternifolia.*

3. The biologically active complex under Claim 1 or Claim 2, wherein said *Eucalyptus spp.* is selected from *E. globulus, E. camaldulensis, E. grandis, E. loxophleba, E. leocoxylon, E. smithii, E. maideni, E. bicostata* or *E. cineria.*

4. The biologically active complex under Claim 3, where *Eucalyptus spp.* contains not less than 70% (v/v) 1,8-cineol.

5. Liquid soap, containing 0.3-0.7 weight % of the biologically active complex under any of Claims 1-4.

6. Shower gel, containing 0.3-0.7 weight % of the biologically active complex under any of Claims 1-4.

7. Scalp care product, containing 0.3-0.7 weight % of the biologically active complex under any of Claims 1-4.

8. Body care product, containing 0.3-0.7 weight % of the biologically active complex under any of Claims 1-4.

9. Cosmetic hygienic product without ethyl alcohol, containing 0.3-0.7 weight % of the biologically active complex under any of Claims 1-4.

10. Non-therapeutic cosmetic use of the biologically active complex under any of Claims 1-4 to regulate the skin microbiota, including inhibition of the growth of the population of opportunistic microorganisms, including bacteria, fungi, maintaining a stable state of the resident microflora.

## Patentansprüche

1. Biologisch aktiver Komplex zur Verwendung in Kosmetikprodukten und/oder Haushaltsreinigern, umfassend:
a) ätherisches Teebaumöl,
b) Alpha-Bisabolol,
c) 1,8-Cineol, gewonnen aus Eukalyptusblättern von *Eucalyptus spp*.,
d) wässrige Lösung aus Zitronensäure und Silbercitrat,
wobei die angegebene wässrige Lösung 19-23 Gew.-% Zitronensäure und 2280-2640 ppm Silbercitrat enthält; und wobei das Massenverhältnis der Komponenten a:b:c:d entsprechend (0,1-0,5):(0,05-0,3):(0,05-0,35):(0,01-0,2) beträgt.

2. Biologisch aktiver Komplex nach Anspruch 1, wobei das ätherische Teebaumöl aus *Melaleuca alternifolia* gewonnen wird.

3. Biologisch aktiver Komplex nach Anspruch 1 oder Anspruch 2, wobei die *Eucalyptus spp.* ausgewählt sind aus *E. globulus, E. camaldulensis, E. grandis, E. loxophleba, E. leocoxylon, E. smithii, E. maideni, E. bicostata* oder *E. cineria*.

4. Biologisch aktiver Komplex nach Anspruch 3, wobei *die Eucalyptus spp.* nicht weniger als 70 % (v/v) 1,8-Cineol enthalten.

5. Flüssigseife, enthaltend 0,3-0,7 Gew.-% des biologisch aktiven Komplexes gemäß einem der Ansprüche 1-4.

6. Duschgel, enthaltend 0,3-0,7 Gew.-% des biologisch aktiven Komplexes gemäß einem der Ansprüche 1 bis 4.

7. Kopfhautpflegeprodukt, enthaltend 0,3-0,7 Gew.-% des biologisch aktiven Komplexes gemäß einem der Ansprüche 1-4.

8. Körperpflegeprodukt, enthaltend 0,3-0,7 Gew.-% des biologisch aktiven Komplexes gemäß einem der Ansprüche 1-4.

9. Kosmetisches Hygieneprodukt ohne Ethylalkohol, enthaltend 0,3-0,7 Gew.-% des biologisch aktiven Komplexes gemäß einem der Ansprüche 1-4.

10. Nichttherapeutische kosmetische Verwendung des biologisch aktiven Komplexes gemäß einem der Ansprüche 1 bis 4 zur Regulierung der Hautmikrobiota, einschließlich der Hemmung des Wachstums der Population opportunistischer Mikroorganismen, einschließlich Bakterien, Pilzen, unter Aufrechterhaltung eines stabilen Zustands der ansässigen Mikroflora.

## Revendications

1. Complexe biologiquement actif conçu pour être utilisé dans des produits cosmétiques et/ou des nettoyants ménagers, comprenant :
a) huile essentielle d'arbre à thé,
b) alpha bisabolol,
c) 1,8-cinéol, dérivé de feuilles d'eucalyptus d'Eucalyptus spp.,
d) solution aqueuse d'acide citrique et de citrate d'argent,
où la solution aqueuse spécifiée contient 19-23 % en poids d'acide citrique et 2 280-2 640 parties par million de citrate d'argent ; et où le rapport massique des composants a:b:c:d est de (0,1-0,5):(0,05-0,3):(0,05-0,35):(0,01-0,2), de manière correspondante.

2. Complexe biologiquement actif selon la revendication 1, dans lequel ladite huile essentielle d'arbre à thé est dérivée de Melaleuca alternifolia.

3. Complexe biologiquement actif selon la revendication 1 ou la revendication 2, dans lequel ledit Eucalyptus spp. est choisi parmi E. globulus, E. camaldulensis, E. grandis, E. loxophleba, E. leocoxylon, E. smithii, E. maideni, E. bicostata ou E. cineria.

4. Complexe biologiquement actif selon la revendication 3, dans lequel Eucalyptus spp. ne contient pas moins de 70 % (v/v) de 1,8-cinéol.

5. Savon liquide, contenant 0,3 à 0,7 % en poids du complexe biologiquement actif selon l'une quelconque des revendications 1 à 4.

6. Gel douche, contenant 0,3 à 0,7 % en poids du complexe biologiquement actif selon l'une quelconque des revendications 1 à 4.

7. Produit de soin du cuir chevelu, contenant 0,3 à 0,7 % en poids du complexe biologiquement actif selon l'une quelconque des revendications 1 à 4.

8. Produit de soins corporels, contenant 0,3 à 0,7 % en poids du complexe biologiquement actif selon l'une quelconque des revendications 1 à 4.

9. Produit cosmétique hygiénique sans alcool éthylique, contenant 0,3 à 0,7 % en poids du complexe biologiquement actif selon l'une quelconque des revendications 1 à 4.

10. Utilisation cosmétique non thérapeutique du complexe biologiquement actif selon l'une quelconque des revendications 1 à 4 pour réguler le microbiote cutané, comprenant l'inhibition de la croissance de la population de micro-organismes opportunistes, y compris des bactéries, des champignons, le maintien d'un état stable de la microflore résidente.
